# EUROPEAN PATENT SPECIFICATION

(11) **EP 0 505 468 B1**
(45) Date of publication and mention of the grant of the patent: **26.04.1995**
(21) Application number: 91901749.1
(22) Date of filing: 11.12.1990
(51) Int. Cl.: C07D 233/84, A61K 31/415, C07D 401/12, C07D 403/12

(54) **IMIDAZOLE DERIVATIVES AND THEIR USE AS ACYL COENZYME-A INHIBITORS**
IMIDAZOL-DERIVATE UND IHRE VERWENDUNG ALS ACYL COENYZM-A INHIBITOREN
DERIVES D'IMIDAZOLE ET LEUR UTILSATION POUR L'INHIBITION DE L'ACYLE COENZYME-A

(30) Priority: 11.12.1989 GB 8927953; 14.08.1990 GB 9017844
(43) Date of publication of application: 30.09.1992
(73) Proprietor: RHONE-POULENC SANTE, 92165 Antony Cédex (FR)
(72) Inventor: BRIDGE, Andrew, William, Dagenham Essex RM10 7XS (GB); HARRIS, Neil, Victor, Dagenham Essex RM10 7XS (GB); LYTHGOE, David, John, Dagenham Essex RM10 7XS (GB); SMITH, Christopher, Dagenham Essex RM10 7XS (GB)
(74) Representative: Bentham, Stephen
(86) International application number: EP9002146
(87) International publication number: WO9109021

(56) References cited:
- EP-A- 0 229 496
- US-A- 4 188 397

## Description

This invention relates to therapeutically useful imidazole derivatives, to processes for their production and to pharmaceutical compositions containing them.

EP-A-229496 discloses compounds of the formula: 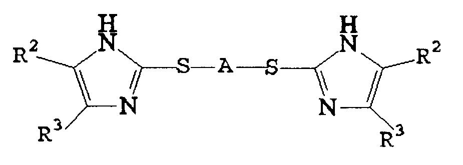
wherein:
A is CH₂CH₂ or CH₂C(O)CH₂; and
R² and R³ are both pyridyl or one of R² and R³ is pyridyl and the other is monosubstituted phenyl wherein said substituent is a halogen atom, and pharmaceutically acceptable salts thereof, for use in treating rheumatoid arthritis.

Th present invention provides a compound of general formula (I), hereinafter depicted, wherein:
A represents a methylene group or a group -CH₂-A'-CH₂-wherein A' represents a direct bond, a linear alkanediyl, alkenediyl or a alkynediyl group containing up to 14 carbon atoms, or a hydroxymethylene group, or A' represents a phenylene group optionally substituted by one to four substituents selected from halogen atoms, alkyl groups (optionally substituted by one or more halogen atoms), alkoxy, alkylthio, alkylamino, carboxy and alkoxycarbonyl groups;
R¹ and R² are the same or different and each represents a hydrogen or halogen atom, an alkyl group (optionally substituted by one or more halogen atoms), or an alkoxy, alkylthio, alkylamino, carboxy or alkoxycarbonyl group;
a and b are the same or different and each represents 0, 1 or 2; and
Het represents a heterocyclic group containing from 5 to 7 ring atoms chosen from carbon, nitrogen, sulphur and oxygen atoms, optionally substituted by one or two substituents independently selected from:-
halogen atoms, alkyl groups (optionally substituted by one or more halogen atoms), hydroxy, amino, alkoxy,alkylthio, alkylamino, carboxy, alkoxycarbonyl and pyridyl groups, and phenyl groups [optionally substituted by one or more substituents selected from halogen atoms, alkyl groups (optionally substituted by one or more halogen atoms), and alkoxy, alkylthio, alkylamino, carboxy and alkoxycarbonyl groups]; and pharmaceutically acceptable salts thereof; wherein all alkyl groups and moieties, unless otherwise indicated, are straight-or branched-chain and contain from 1 to 5 carbon atoms; and halogen atoms, unless otherwise indicated, are fluorine, chlorine, bromine or iodine atoms;
for use in the preparation of a pharmaceutical composition for the treatment of conditions which can be ameliorated by administration of an inhibitor of acyl coenzyme-A:cholesterol-O-acyl transferase such as atherosclerosis, hyperlipidaemia, cholesterol ester storage disease and atheroma in vein grafts.

The present invention also provides a pharmaceutical composition which comprises a compound of general formula (I), or a pharmaceutically acceptable salt thereof, as hereinbefore defined provided that:-
when R¹ and R² are identical and each represents a fluorine, chlorine or bromine atom or a trifluoromethyl, methoxy or methylthio group in the para-position and A represents a methylene, 1,2-ethanediyl, 1,3-propanediyl, 2-hydroxy-1,3-propanediyl or 1,4-butanediyl group, then Het represents other than a group of general formula (VIII), as hereinafter depicted, wherein R³ represents a hydrogen atom or a methyl group and the symbols R⁴ are identical and each represents a fluorine, chlorine or bromine atom or a trifluoromethyl, methoxy or methylthio group in the para-position;
in association with a pharmaceutically acceptable carrier or coating.

The present invention also provides new imidazole derivatives of general formula (I), and pharmaceutically acceptable salts thereof, as hereinbefore defined subject to the provisos that:-
1) when R¹ and R² are identical and each represents a fluorine, chlorine or bromine atom or a trifluoromethyl, methoxy or methylthio group in the para-position and A represents a methylene, 1,2-ethanediyl, 1,3-propanediyl, 2-hydroxy-1,3-propanediyl or 1,4-butanediyl group, then Het represents other than a group of formula (VIII), as hereinafter depicted, wherein R³ represents a hydrogen atom or a methyl group and the symbols R⁴ are identical and each represents a fluorine, chlorine or bromine atom or a trifluoromethyl, methoxy or methylthio group in the para-position; and
2) when A represents a 1,3-propanediyl or 2-hydroxy-1,3-propanediyl group, R¹ and R² both represent hydrogen atoms, and a and b both represent zero, then Het represents other than a group of formula (VIII) wherein R³ represents a hydrogen atom and the symbols R₄ both represent hydrogen atoms. Especially preferred imidazole derivatives of the present in formula (I) at least one of the symbols has a value selected from the following invention are those in which:-
   (i) A represents a group -CH₂-A'-CH₂- wherein A' represents a direct bond, a linear alkanediyl, alkenediyl or alkynediyl group containing up to 7 carbon atoms (more especially a methylene group or a linear alkanediyl group containing 2, 3, 4 or 7 carbon atoms or a vinylene or ethynylene group) or a hydroxymethylene group, or a substituted or, more especially, unsubstituted phenylene group;
   (ii) R¹ and R² are the same or different and each represents a hydrogen or halogen atom, or an alkyl or alkoxy group, more especially a hydrogen atom, or a fluorine atom or a methyl or methoxy group, preferably in the 4-position; and
   (iii) Het represents an optionally substituted 5- or 6-membered heterocyclic group containing up to 4 nitrogen atoms, for example an optionally substituted pyrimidinyl (preferably pyrimidin-2-yl), pyridyl (preferably pyrid-2-yl or pyrid-4-yl), imidazolyl (preferably imidazol-2-yl), triazolyl (preferably 1,2,4-triazol-3-yl) or tetrazolyl (preferably tetrazol-5-yl) group. Preferred substituents on the group Het are alkyl (preferably methyl), amino, pyridyl (e.g. pyrid-2-yl), and optionally substituted phenyl groups;
      the other symbol being as hereinbefore defined, and their pharmaceutically acceptable salts.
Preferred salts are acid addition salts, such as, for example, the hydrochlorides and maleates.

The compounds of formula (I), as hereinbefore defined, are inhibitors of acyl coenzyme-A:cholesterol-O-acyl transferase (ACAT;EC 2.3.1.26). They are therefore of value as anti-atherosclerotic agents and have utility in the treatment of atherosclerosis, hyperlipidaemia, cholesterol ester storage disease and atheroma in vein grafts.

In assays performed in vitro, microsomes (prepared from the livers of rats fed a diet supplemented with 0.5%w/w cholesterol and 0.25%w/w cholic acid for 7 days) were incubated with radiolabelled oleoyl-CoA in the presence of compounds according to the invention at a concentration (in »g/m1) as shown in Table 1. The degree of ACAT inhibition produced is shown in Table 1.

In in vivo tests, using rats fed on a similar diet to that above and further supplemented by the indicated amount (%w/w) of test compound, compounds according to the invention inhibited increases in plasma cholesterol level, measured after 3 days, relative to control animals fed on the cholesterol supplemented diet without the drug, as shown in Table 1.

**Table 1**

| Compound | In-vitro | | In-vivo | |
|---|---|---|---|---|
| | Concentration | % Inhibition | Dose | % Reduction |
| A | 0.5 | 88 | 0.03 | 7 |
| B | 0.5 | 90 | 0.03 | 91 |
| C | 0.5 | 93 | 0.03 | 66 |
| D | | | 0.03 | 88 |
| E | 1.0 | 88 | | |
| F | 1.0 | 96 | 0.03 | 72 |
| G | 0.5 | 80 | | |
| H | 0.5 | 90 | 0.03 | 108 |
| I | 0.5 | 84 | 0.01 | 5 |
| J | 0.5 | 84 | 0.01 | 17 |
| K | 0.5 | 77 | 0.01 | 4 |
| L | 0.5 | 19 | | |
| M | 0.5 | 95 | 0.01 | 107 |
| N | 0.5 | 94 | 0.03 | 98 |
| O | 0.5 | 23 | | |
| P | 0.5 | 97 | | |
| Q | 0.5 | 98 | 0.01 | 74 |
| R | 0.5 | 95 | | |
| S | 0.5 | 77 | | |
| T | 0.5 | 91 | | |

Compounds within the scope of general formula (I), wherein A represents a methylene, 1,2-ethanediyl, 1,3-propanediyl, 2-hydroxy-1,3-propanediyl or 1,4-butanediyl group, R¹ and R² are identical and each represents a fluorine, chlorine or bromine atom or a trifluoromethyl, methoxy or methylthio group in the para-position, a and b are the same or different and each represents 0, 1 or 2, and Het represents a group of general formula (VIII), as hereinafter depicted, wherein R³ represents a hydrogen atom or a methyl group and the symbols R⁴ are identical and each represents a fluorine, chlorine or bromine atom or a trifluoromethyl, methoxy or methylthio group in the para-position and their pharmaceutically acceptable salts, alone and in association with pharmaceutically acceptable carriers or coatings, have been disclosed in United States Patent Specification No. 4188397.

However, in that specification, those compounds and their pharmaceutical compositions are described as regulating cell-mediated immunity and having anti-arthritic activity. Nowhere in that specification is there any suggestion that the compounds or their pharmaceutical compositions could be useful as imhibitors of acyl coenzyme-A:cholesterol-O-acyl transferase or in the treatment of conditions such as atherosclerosis, hyperlipidaemia, cholesterol ester storage disease or atheroma in vein grafts.

Compounds within the scope of general formula (I), wherein A represents a 1,3-propanediyl or 2-hydroxy-1,3-propanediyl group, R¹ and R² both represent hydrogen atoms, a and b both represent zero and Het represents a group of formula (VIII) wherein R³ represents a hydrogen atom and the symbols R⁴ both represent hydrogen atoms, have been disclosed by Bagry et al., Dopovidi AN URSR, (9), 801-804, 1975.

However, in that paper, no biological activity nor utility of those compounds is described, nor any pharmaceutically acceptable composition, and nowhere in that paper is there any suggestion that the compounds could be useful as inhibitors of acyl coenzyme-A:cholesterol-O-acyl transferase or in the treatment of conditions such as atherosclerosis, hyperlipidaemia, cholesterol ester storage disease or atheroma in vein grafts.

Accordingly, the invention also provides a compound of general formula (I), or a pharmaceutically acceptable salt thereof, as hereinbefore defined, provided that when R¹ and R² are identical and each represents a fluorine, chlorine or bromine atom or a trifluoromethyl, methoxy or methylthio group in the para position and A represents a methylene, 1,2-ethanediyl, 1,3-propanediyl, 2-hydroxy-1,3-propanediyl or 1,4-butanediyl group then Het represents other than a group of formula, as hereinafter depicted, wherein R³ represents a hydrogen atom or a methyl group and the symbols R⁴ are identical and each represents a fluorine, chlorine or bromine atom or a trifluoromethyl, methoxy or methylthio group in the para position, for use in a new method of treatment of the human or animal body by therapy, of condition which can be ameliorated by administration of an inhibitor of acyl coenzyme-A:cholesterol-O-acyl transferase such as atherosclerosis, hyperlipidaemia, cholesterol ester storage disease and atheroma in vein grafts.

Thus, the utilities disclosed in the present specification are entirely unexpected.

Important compounds encompassed by the various aspects of the invention include:
A 2-[3-(Pyrimidin-2-ylthio)propylthio]-4,5-diphenylimidazole
B 2-[3-(Pyrid-4-ylthio)propylthio]-4,5-diphenylimidazole
C 2-[3-(1-Methylimidazol-2-ylthio)propylthio]-4,5-diphenylimidazole
D 2-[3-(1-Methylimidazol-2-ylthio)propylthio]-4,5-diphenylimidazole dihydrochloride
E 1,2-Bis(4,5-diphenylimidazol-2-ylthio)ethane
F 1,3-Bis(4,5-diphenylimidazol-2-ylthio)propane
G 2-[3-(4,6-Dimethylpyrimidin-2-ylthio)propylthio]-4,5-diphenylimidazole
H 2-[3-(1-Methylimidazol-2-ylsulphonyl)propylthio]-4,5-diphenylimidazole
I 2-[3-(4-Methyl-1,2,4-triazol-3-ylthio)propylthio]-4,5-diphenylimidazole
J 2-[3-(1-Methyltetrazol-5-ylthio)propylthio]-4,5-diphenylimidazole
K 1-(4,5-Diphenylimidazol-2-ylthio)-3-[(4,5-dipyrid-2-yl)imidazol-2-ylthio]propane dihydrochloride
L 2-[3-(1-Methyltetrazol-5-ylsulphonyl)propylthio]-4,5-diphenylimidazole
M 2-[3-(Imidazol-2-ylthio)propylthio]-4,5-diphenylimidazole
N 2-[4-(1-Methylimidazol-2-ylthio)butylthio]-4,5-diphenylimidazole
O 2-[4-(1-Methylimidazol-2-ylsulphonyl)butylsulphonyl]-4,5-diphenylimidazole
P 1,4-Bis(4,5-diphenylimidazol-2-ylthio)butane
Q E-1,4-Bis(4,5-diphenylimidazol-2-ylthio)but-2-ene
R 2-[6-(1-Methylimidazol-2-ylthio)hexylthio]-4,5-diphenylimidazole
S 1,9-Bis(4,5-diphenylimidazol-2-ylthio)nonane
T 2-[9-(1-Methylimidazol-2-ylthio)nonylthio]-4,5-diphenylimidazole
AA 1,2-bis(4,5-diphenylimidazol-2-ylthiomethyl)benzene
AB 1,3-bis(4,5-diphenylimidazol-2-ylthiomethyl)benzene
AC 1,4-bis(4,5-diphenylimidazol-2-ylthiomethyl)benzene
AD 1,3-bis(4,5-diphenylimidazol-2-ylthio)propan-2-ol
AE 4,5-diphenyl-2-[3-(1-methylimidazol-2-ylthio)-2-hydroxypropylthio]imidazole
BA 4,5-diphenyl-2-[3-(1-methylimidazol-2-ylthio)propylthio]imidazole dimaleate
BB 4,5-Diphenyl-2-[3-(3-amino-1,2,4-triazol-5-ylthio)propylthio]imidazole
BC 4,5-Diphenyl-2-[3-(pyrid-2-ylthio)propylthio]imidazole
BD E-4,5-Diphenyl-2-[4-(1-methylimidazol-2-ylthio)but-2-enylthio]imidazole
BE 4,5-Diphenyl-2-[3-(pyrimidin-2-ylsulphonyl)propylthio]imidazole
BF 4,5-Diphenyl-2-[4-(pyrimidin-2-ylsulphonyl)butylthio]imidazole
BG 4,5-Diphenyl-2-[3-(pyrid-2-ylsulphinyl)propylthio]imidazole
BH 4,5-Diphenyl-2-[4-(pyrimidin-2-ylthio)butylthio]imidazole
BI 1,6-Bis(4,5-diphenylimidazol-2-ylthio)hexane
BJ 4,5-Bis(4-methylphenyl)-2-[3-(1-methylimidazol-2-ylthio)propylthio]imidazole
BK 4,5-Bis(4-methoxyphenyl)-2-[3-(pyrimidin-2-ylsulphonyl)propylthio]imidazole
BL 4,5-Diphenyl-2-[4-(imidazol-2-ylthio)butylthio]imidazole
BM 4,5-Diphenyl-2-[3-(pyrid-4-ylsulphonyl)propylthio]imidazole
BN 4,5-Diphenyl-2-[3-(pyrid-2-ylsulphonyl)propylthio]imidazole
BO 4,5-Diphenyl-2-[4-(pyrid-2-ylthio)butylthio]imidazole
BP 1,5-Bis(4,5-diphenylimidazol-2-ylthio)pentane
BQ 1,4-Bis(4,5-diphenylimidazol-2-ylthio)but-2-yne
BR 4,5-Diphenyl-2-[4-(1-methylimidazol-2-ylthio)but-2-ynylthio]imidazole
BS 4,5-Bis(4-fluorophenyl)-2-[3-(1-methylimidazol-2-ylthio)propylthio]imidazole
BT 4,5-Bis(4-methoxyphenyl)-2-[4-(pyrimidin-2-ylsulphonyl)butylthio]imidazole
BU 4,5-Diphenyl-2-[4-(pyrid-2-ylsulphonyl)butylthio]imidazole
BV 4,5-Diphenyl-2-[4-(pyrid-2-ylsulphinyl)butylthio]imidazole
BW 4,5-Diphenyl-2-{3-[4(5)-methylimidazol-2-ylthio]propylthio}imidazole
BX 4,5-Diphenyl-2-[5-(imidazol-2-ylthio)pentylthio]imidazole
BY 4,5-Diphenyl-2-[3-(imidazol-2-ylthio)propylthio]imidazole dihydrochloride
BZ 4,5-Diphenyl-2-[3-(imidazol-2-ylthio)propylthio]imidazole dimaleate
CA Z-1,4-Bis(4,5-diphenylimidazol-2-ylthio)but-2-ene
CB 4,5-Diphenyl-2-[4-(pyrid-2-ylsulphonyl)butylthio]imidazole hydrochloride
CC 4,5-Diphenyl-2-[4-(imidazol-2-ylsulphonyl)butylthio]imidazole.

The letters A to CC are allocated to compounds for easy reference throughout this specification.

Compounds of formula (I) can be prepared by the application or adaptation of known methods, by which is meant methods used heretofore or described in the literature.

Thus, according to a feature of the invention, the novel imidazole derivatives of general formula (I), wherein A, R¹, R², b and Het are as hereinbefore defined and a represents zero, are prepared by reacting a salt of general formula (II), as hereinafter depicted, wherein R¹ and R² are as hereinbefore defined and M represents an alkali metal, preferably sodium or potassium, atom, with a compound of formula:

Z¹-A-S(O)_{b}-Het (III)

wherein A, b and Het are as hereinbefore defined and Z¹ represents a chlorine, bromine, or iodine atom, or an alkyl- or aryl-sulphonyloxy group, for example a methanesulphonyloxy or 4-toluenesulphonyloxy group.

According to a further feature of the invention, compounds of formula (I), wherein A, R¹, R², a and Het are as hereinbefore defined and b represents zero, are prepared by reacting a salt of formula:

Het-S⁻ M⁺ (V)

wherein Het and M are as hereinbefore defined, with a compound of general formula (IV), hereinafter depicted, wherein A, R¹, R², and a are as hereinbefore defined and Z² represents a chlorine, bromine, or iodine atom, or an alkyl- or aryl-sulphonyloxy group, for example a methanesulphonyloxy or 4-toluenesulphonyloxy group.

According to a further feature of the invention, compounds of formula (I), wherein A, R¹ and R² are as hereinbefore defined, a and b are both zero and Het represents a substituted or unsubstituted 4,5-diphenylimidazol-2-yl group identical to that at the other end of the molecule, [i.e. a compound of general formula (VI), hereinafter depicted, wherein A, R¹ and R² are as hereinbefore defined], are prepared by reacting a salt of formula (II) with a compound of formula:

Z¹-A-Z² (VII)

wherein A, Z¹ and Z² are as hereinbefore defined.

According to a further feature of the invention, compounds of formula (I), wherein A, R¹, R² and Het are as hereinbefore defined and a and b are both zero, are prepared by reacting a salt of formula (II) and a salt of formula (V) with a compound of formula (VII) wherein the various symbols are as hereinbefore defined.

Each of the above reactions between compounds of formulae (II) and (III), (V) and (IV), (II) and (VII), and (II), (V) and (VII) may be performed in an inert solvent, such as dimethylformamide or tetrahydrofuran, at temperatures between room temperature and the reflux temperature of the reaction mixture.

According to a further feature of the present invention, compounds of general formula (I) are prepared by the interconversion of other compounds of general formula (I). For example, compounds containing alkoxycarbonyl groups can be prepared by the alkylation of compounds containing carboxy groups by the application or adaptation of known methods of alkylation, and compounds containing carboxy groups can be prepared by the hydrolysis of compounds containing alkoxycarbonyl groups by the application or adaptation of known methods of hydrolysis.

According to a further feature of the invention, compounds of formula (I), wherein A, R¹, R² and Het are as hereinbefore defined and at least one of a and b represents 1 or 2, are prepared by the oxidation of a compound of formula (I), wherein A, R¹, R² and Het are as hereinbefore defined and a and/or b is less than in the desired product.

The oxidation may be performed by using a conventional oxidant, such as a percarboxylic acid (e.g. m-chloroperbenzoic acid), in an inert solvent, such as dichloromethane, at or below room temperature.

By the term "pharmaceutically acceptable salts" as used in this specification is meant salts the anions or cations of which are relatively innocuous to the animal organism when used in therapeutic doses so that the beneficial pharmaceutical properties of the parent compounds of general formula (I) capable of forming salts are not vitiated by side-effects ascribable to those anions or cations.

Suitable acid addition salts for use in pharmaceuticals may be selected from salts derived from inorganic acids, for example hydrochlorides, hydrobromides, phosphates, sulphates and nitrates, and organic acids, for example oxalates, lactates, tartrates, acetates, salicylates, citrates, propionates, succinates, fumarates, maleates, methylene-bis-β-hydroxynaphthoates, gentisates and di-p-toluoyltartrates.

Suitable salts with bases include alkali metal (e.g. sodium and potassium), alkaline earth metal (e.g. calcium and magnesium), ammonium and amine (e.g. diethanolamine, triethanolamine, octylamine, morpholine and dioctylmethylamine) salts.

According to a further feature of the invention, salts of compounds of formula (I) are prepared by reaction of the parent compounds of formula (I) with the appropriate acid or base, by the application or adaptation of known methods.

As well as being useful in themselves as active compounds, salts of compounds of formula (I) are useful for the purposes Of purification of the parent compounds of formula (I), for example by exploitation of the solubility differences between the salts and the parent compounds, by techniques well known to those skilled in the art.

The parent compounds of formula (I) can be regenerated from their salts by the application or adaptation of known methods.

For example, parent compounds of general formula (I) con be regenerated from their acid addition salts by treatment with an alkali, e.g. aqueous sodium bicarbonate solution or aqueous ammonia solution.

Parent compounds of general formula (I) can be regenerated from their salts with bases by treatment with an acid, e.g. dilute hydrochloric acid.

In this specification reference to compounds of formula (I) is intended to include reference to their pharmaceutically acceptable salts, where the context so permits.

The starting materials and intermediates can be prepared by the application or adaptation of known methods.

Thus, compounds of formulae (III) and (IV), wherein A, R¹, R², Z¹, Z² and Het are is hereinbefore defined and a or b represents zero, can be made by the reaction of salts of formulae (II) or (V), wherein R¹, R², Het and M are as hereinbefore defined, with compounds of general formula (VII), as hereinbefore defined, Z¹ and Z² preferably being different, by the application or adaptation of conditions similar to those described above in respect of the reactions between compounds of formulae (II) and (III), (V) and (IV), and (II) and (VII), such that only one of Z¹ and Z² is displaced.

Compounds of formulae (III) or (IV), wherein A, R¹, R², Z¹, Z² and Het are as hereinbefore defined and a or b represents zero, can be oxidised to the corresponding compounds, wherein a or b is greater than in the starting material, by the application or adaptation of conditions similar to those described above in respect of the oxidation of compounds of formula (I).

Compounds of formula (I) can be purified by the usual physical means, for example by crystallisation or chromatography.

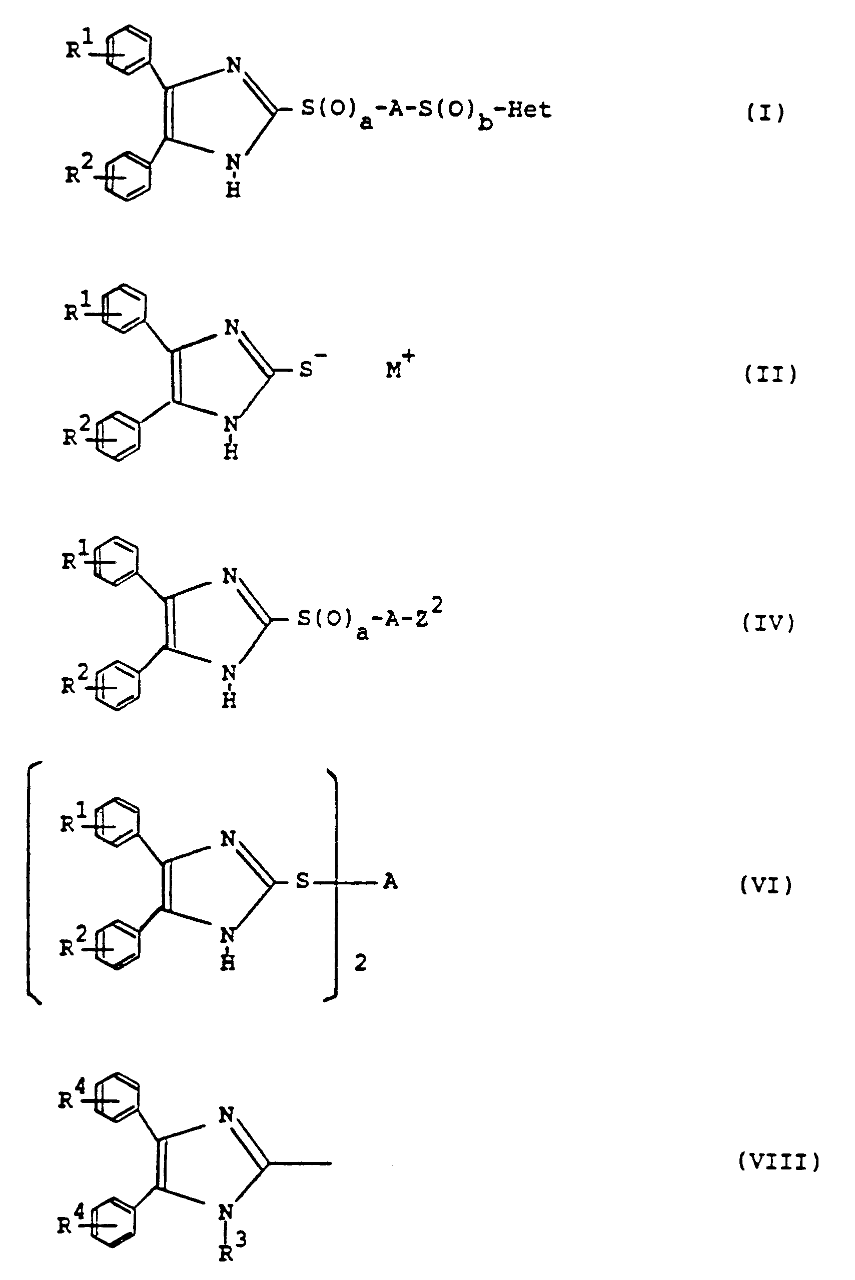

The following Examples illustrate the preparation of compounds encompassed by the various aspects of the invention and the Reference Examples illustrate the preparation of certain intermediates.

### EXAMPLE 1

### Compounds A, B and C

i) Potassium t-butoxide (10.0g) was added to 2-mercaptopyrimidine (10.0g) in dimethylformamide (DMF) (100ml) and the mixture was stirred at ambient temperature for 15 minutes, then 1-chloro-3-iodopropane (9.6ml; 18.23g) was added and the mixture stirred for a further 2 hours. A solution prepared by adding potassium t-butoxide (5.0g) to 4,5-diphenylimidazole-2-thiol (11.25g) in DMF (100ml) was then added and the resulting mixture was stirred overnight. The reaction mixture was concentrated under reduced pressure to leave an orange-red oil. Water (100ml) was added and the product was extracted into ethyl acetate (3x150ml). The organic solution was dried over magnesium sulphate and then concentrated under reduced pressure to leave a red oil. Chromatography on silica, eluting with dichloromethane / ethyl acetate (85:15) yielded 2-[3-(pyrimidin-2-ylthio)propylthio]-4,5-diphenylimidazole (13.0g) in the form of a white solid, after recrystallisation from aqueous ethanol, mp 110-115°C; [Found:- C,65.2;H,4.91;N,14.1;S,16.1%; Calculated for C₂₂H₂₀N₄S₂:- C,65.32;H,4.98;N,13.83;S,15.83%].
ii) By proceeding in a similar manner, but replacing the 2-mercaptopyrimidine by 4-mercaptopyridine and using dichloromethane / ethyl acetate (75:25) as eluent, there was prepared 2-[3-(pyrid-4-ylthio)propylthio]-4,5-diphenylimidazole, in the form of a pale yellow solid, after trituration with diethyl ether, mp 125-127°C; [Found:- C,68.0;H,5.15;N,10.4; S,16.3%; Calculated for C₂₃H₂₁N₃S₂:- C,68.45;H,5.25; N,10.41;S,15.89%].
iii) By proceeding in a similar manner, but replacing the 2-mercaptopyrimidine by 1-methyl-2-mercaptoimidazole and using dichloromethane / ethyl acetate (75:25) and then ethyl acetate as eluents, there was prepared 2-[3-(1-methylimidazol-2-ylthio)propylthio)-4,5-diphenylimidazole, in the form of a white powder, after trituration with diisopropyl ether, mp 142°C; [Found:- C,65.2;H,5.62;N,13.9;S 16.1%; Calculated for C₂₂H₂₂N₄S₂:- C,64.99;H,5.45;N,13.78; S,15.77%].

### EXAMPLE 2

### Compound D

A solution of 1 equivalent of 2-[3-(1-methylimidazol-2-ylthio)propylthio]-4,5-diphenylimidazole in ethanol was added to a solution of hydrogen chloride in ethanol prepared by adding 3 equivalents of acetyl chloride to ice-cooled ethanol. The solution was concentrated under reduced pressure and the residue was triturated with diethyl ether to yield 2-[3-(1-methylimidazol-2-ylthio)propylthio]-4,5-diphenylimidazole dihydrochloride as a white solid; [Found:- C,51.3; H,5.5;N,11.0;Cl,14.0;S,12.3%; Calculated for C₂₂H₂₂N₄S₂.2HCl.2H₂O:- C,51.26;H,5.44;N,10.87;Cl,13.76; S,12.43%].

### EXAMPLE 3

### Compounds E and F

i) Sodium hydride (0.9g; 80% dispersion in mineral oil) was carefully added to a stirred suspension of 4,5-diphenylimidazole-2-thiol (7.5g) in dry tetrahydrofuran (THF) (300ml) to give a yellow solution. 1,2-Dichloroethane (1.5g) was added and the solution was heated at reflux for 26 hours, then poured into water. The solid was collected and washed with water and then stirred with methanol and dichloromethane (1:1). The solid was collected, washed with diethyl ether, then dissolved in DMF, diluted with diethyl ether and left to recrystallise to give 1,2-bis(4,5-diphenylimidazol-2-ylthio)ethane (1.18g) as a white powder, mp 310°C; (Found:- C,72.4;H,4.91; N,10.6;S,12.1%.; Calculated for C₃₂H₂₆N₄S₂:- C,72.42; H,4.94;N,10.56;S,12.08%].
ii) By proceeding in a similar manner, but replacing the sodium hydride by potassium t-butoxide, replacing the THF by DMF and replacing the 1,2-dichloroethane by 1-chloro-3-iodopropane, stirring at ambient temperature overnight and purifying the crude product by chromatography on silica, eluting with dichloromethane / ethyl acetate (3:2), there was prepared 1,3-bis(4,5-diphenylimidazol-2-ylthio)propane, as a white solid, after crystallisation from ethyl acetate, mp 240°C; [Found:- C,72.9;H,5.2; N,10.1;S,11.3%; Calculated for C₃₃H₂₈N₄S₂:- C,72.76; H,5.18;N,10.29;S,11.77%].

### EXAMPLE 4

### Compound G

By proceeding in a similar manner to that described in Example 1 but replacing the 2-mercaptopyrimidine by 4,6-dimethyl-2-mercaptopyrimidine and using dichloromethane / ethyl acetate (4:1) as eluent, there was prepared 2-[3-(4,6-dimethylpyrimidin-2-ylthio)propylthio]-4,5-diphenylimidazole, in the form of a pale green solid, after trituration with diethyl ether, mp 122-124°C; [Found:- C,66.5;H,5.54;N,12.9; S,14.8%; Calculated for C₂₄H₂₄N₄S₂:- C,66.63;H,5.59; N,12.95;S,14.82%].

### EXAMPLE 5

### Compound H

A mixture of 4,5-diphenylimidazole-2-thiol (5.04g) and potassium tert-butoxide (2.35g) in dry dimethylformamide (DMF) (75ml) was stirred for 15 minutes to give a yellow suspension. A solution of 1-methyl-2-(3-chloropropylsulphonyl)imidazole (4.68g) in dry DMF (10ml) was added and the resulting mixture was stirred overnight at ambient temperature. The mixture was concentrated under reduced pressure and the residue was dissolved in ethyl acetate and twice washed with water. After drying over magnesium sulphate, the solution was concentrated under reduced pressure. Chromatography of the residue on silica, eluting with ethyl acetate afforded 2-[3-(1-methylimidazol-2-ylsulphonyl)propylthio]-4,5-diphenylimidazole, in the form of a white solid after crystallisation from ethyl acetate, mp 143-144°C; (Found:- C,59.8;H,5.04;N,12.7; S,14.6%; Calculated for C₂₂H₂₂N₄O₂S₂:- C,60.25;H,5.06; N,12.78;S,14.62%].

### EXAMPLE 6

### Compounds I and J

i) By proceeding in a similar manner to that described in Example 1, but replacing the initial use of potassium tert-butoxide by sodium hydride (80% dispersion in mineral oil) and replacing the 2-mercaptopyrimidine by 4-methyl-2-mercapto-1,2,4-triazole, stirring overnight after the addition of 1-chloro-3-iodopropane and using ethyl acetate followed by ethyl acetate / methanol (4:1) as eluents, there was prepared 2-[3-(4-methyl-1,2,4-triazol-3-ylthio)propylthio]-4,5-diphenylimidazole, in the form of a white solid from ethyl acetate, mp 141-142°C; [Found:-C,61.5;H,5.0;N,17.5;S,15.6%; Calculated for C₂₁H₂₁N₅S₂:- C,61.89;H,5.19;N,17.18;S,15.74%].
ii) By proceeding in a similar manner to that described in Example 1, but omitting the initial use of potassium tert-butoxide and replacing the 2-mercaptopyrimidine by the sodium salt of 1-methyl-5-mercaptotetrazole, stirring overnight after the addition of 1-chloro-3-iodopropane and using ethyl acetate / dichloromethane (1:2) as eluent, there was prepared 2-[3-(1-methyltetrazol-5-ylthio)propylthio]-4,5-diphenylimidazole in the form of a white solid from ethyl acetate, mp 131°C; [Found:- C,58.8;H,4.86; N,21.0;S,15.7%; Calculated for C₂₀H₂₀N₆S₂:- C,58.79; H,4.93;N,20.57;S,15.70%].

### EXAMPLE 7

### Compound K

Potassium tert-butoxide (0.56g) was added to a solution of 2-mercapto-4,5-dipyrid-2-ylimidazole (1.27g) in dry DMF (30ml). After stirring for 15 minutes, a solution of 2-(3-chloropropylthio)-4,5-diphenylimidazole (1.64g) in dry DMF (10ml) was added. The resulting mixture was stirred overnight at ambient temperature and then concentrated under reduced pressure. The residue was taken up in ethyl acetate (100ml) and twice washed with water, then dried over magnesium sulphate. Chromatography on silica, eluting with ethyl acetate, afforded a yellow solid which was dissolved in ethanol (50ml) and treated with a solution of hydrogen chloride in ethanol. After stirring for 30 minutes, the solution was concentrated under reduced pressure and the residue was triturated with diethyl ether to give 1-(4,5-diphenylimidazol-2-ylthio)-3-[(4,5-dipyrid-2-yl)imidazol-2-ylthio]propane dihydrochloride, as a yellow solid; [Found:- C,57.7; H,5.1;N,12.7;Cl,10.6;S,9.6%; Calculated for C₃₁H₂₆N₆S₂.2HCl.1½H₂O:- C,57.59;H,4.83;N,13.0;Cl,10.97; S,9.92%].

### EXAMPLE 8

### Compound L

By proceeding in a similar manner to that described in Example 5, but replacing the 1-methyl-2-(3-chloropropylsulphonyl)imidazole with 1-methyl-5-(3-chloropropylsulphonyl)tetrazole, there was prepared 2-[3-(1-methyltetrazol-5-ylsulphonyl)propylthio]-4,5-diphenylimidazole, as a crystalline solid from ethyl acetate, mp 157-158°C; [Found:- C,54.1;H,4.44;N,19.2; S,14.5%; Calculated for C₂₀H₂₀N₆O₂S₂:- C,4.53;H,4.58; N,19.08;S,14.56%].

### EXAMPLE 9

### Compounds M and N

i) By proceeding in a similar manner to that described in Example 1, but replacing the 2-mercaptopyrimidine by 2-mercaptoimidazole and using ethyl acetate as eluent, there was prepared 2-[3-(imidazol-2-ylthio)propylthio]-4,5-diphenylimidazole, in the form of a white solid, after crystallisation from ethyl acetate, mp 163-164°C; [Found:- C,63.9;H,5.0;N,14.4; S,16.2%; Calculated for C₂₁H₂₀N₄S₂:- C,64.25;H,5.14; N,14.27;S,16.34%.
ii) By proceeding in a similar manner to that described in Example 1, hut replacing the 2-mercaptopyrimidine by 1-methyl-2-mercaptoimidazole and replacing the 1-chloro-3-iodopropane with 1-chloro-4-iodobutane and using ethyl acetate as eluent, there was prepared 2-[4-(1-methylimidazol-2-ylthio)butylthio]-4,5-diphenylimidazole, as a solid after trituration with diethyl ether and recrystallisation from ethyl acetate, mp 111-112°C; [Found:- C,65.9;H,5.73;N,13.3; S,15.3%; Calculated for C₂₃H₂₄N₄S₂:- C,65.68;H,5.75; N,13.32;S,15.25%].

### EXAMPLE 10

### Compound O

A suspension of 2-[4-(1-methylimidazol-2-ylthio)butylthio]-4,5-diphenylimidazole (2.5g) in dichloromethane (150ml) was treated with m-chloroperbenzoic acid (7.2g; 72% mixture with water). The mixture was stirred overnight at ambient temperature and then an aqueous solution of sodium metabisulphite was added. The organic phase was washed twice with aqueous sodium bicarbonate and then with water and dried over magnesium sulphate. Concentration under reduced pressure left a foam which was crystallised from ethyl acetate to give 2-[4-(1-methylimidazol-2-ylsulphonyl)butylsulphonyl]-4,5-diphenylimidazole, mp 149-150°C; [Found:- C,57.0;H,5.0;N,11.6;S,13.2%; Calculated for C₂₃H₂₄N₄O₄S₂:- C,57.00;H,5.00;N,11.57; S,13.23%].

### EXAMPLE 11

### Compounds P and Q

i) By proceeding in a similar manner to that described in Example 3, but replacing the 1,2-dichloroethane by 1-chloro-4-bromobutane and purifying the crude product (which was isolated by extraction into ethyl acetate) by chromatography on silica, eluting with dichloromethane / methanol (19:1), there was prepared 1,4-bis(4,5-diphenylimidazol-2-ylthio)butane, as a colourless solid, mp 230-232°C; [Found:- C,72.8; H,5.4;N,10.01;S,11.6%; Calculated for C₃₄H₃₀N₄S₂:-C,73.08;H,5.41;N,10.03;S,11.48%].
ii) By proceeding in a similar manner, but replacing the 1,2-dichloroethane by 1,4-dibromobut-2-ene and purifying the crude product (which was isolated by extraction into ethyl acetate) by crystallisation from dichloromethane followed by recrystallisation from isopropanol, there was prepared E-1,4-bis(4,5-diphenylimidazol-2-ylthio)but-2-ene, in the form of a colourless solid, mp 233-235°C; [Found:-C,73.4;H,5.16;N,10.1;S,11.6%; Calculated for C₃₄H₂₈N₄S₂:- C,73.35;H,5.07;N,10.06;S,11.52%].

### EXAMPLE 12

### Compound R

By proceeding in a similar manner to that described in Example 1, but replacing the original use of potassium tert.-butoxide by sodium hydride (80% dispersion in mineral oil), replacing the 2-mercaptopyrimidine by 1-methyl-2-mercaptoimidazole, replacing the 1-chloro-3-iodopropane by 1-bromo-6-chlorohexane and using ethyl acetate / dichloromethane (7:3) as eluent, there was prepared 2-[6-(1-methylimidazol-2-ylthio)hexylthio]-4,5-diphenylimidazole, in the form of a white solid, after recrystallisation from ethyl acetate, m.p. 107-108°C; [Found:- C,66.7;H,6.3;N,12.6; S,13.9%; Calculated for C₂₅H₂₈N₄S₂:- C,66.93;H,6.29; N,12.49;S,14.29%].

### EXAMPLE 13

### Compound S

By proceeding in a similar manner to that described in Example 3 ii), but replacing the 1-chloro-3-iodopropane by 5 equivalents of 1,9-dibromononane and purifying the crude product by chromatography on silica, eluting with diethyl ether / cyclohexane (15:85) followed by diethyl ether, there were prepared:-
i) from the ether / cyclohexane eluate, 2-(9-bromononylthio)-4,5-diphenylimidazole, as a white solid, after recrystallisation from cyclohexane, m.p. 96-97°C; [Found:- C,62.6;H,6.3;N,6.0;Br,17.4;S,7.0%; Calculated for C₂₄H₂₉BrN₂S:- C,63.01;H,6.39;N,6.12; Br,17.47;S,7.01%]; and
ii) from the ether eluate, 1,9-bis(4,5-diphenylimidazol-2-ylthio)nonane, as a white solid, from ethyl acetate, m.p.199-200°C; [Found:- C,73.5;H,6.4;N,8.7; S,10.1%; Calculated for C₃₉H₄₀N₄S₂.0.3C₂H₅O₂CCH₃:-C,73.68;H,6.52;N,8.55;S,9.79%].

### EXAMPLE 14

### Compound T

By proceeding in a similar manner to that described in Example 7, but replacing the 2-mercapto-4,5-dipyrid-2-ylimidazole by 1-methyl-2-mercaptoimidazole, replacing the 2-(3-chloropropylthio)-4,5-diphenylimidazole by 2-(9-bromononylthio)-4,5-diphenylimidazole (prepared as in Example 13) and eluting with dichloromethane / methanol (3:1), there was prepared 2-[9-(1-methylimidazol-2-ylthio)nonylthio]-4,5-diphenylimidazole, as a white solid, after trituration with petrol (b.p. 40-60°C), m.p. 101-102°C; [Found:- C,68.4;H,6.95;N,11.5;S,13.0%; Calculated for C₂₈H₃₄N₄S₂:- C,68.53;H,6.98;N,11.42;S,13.07%].

### EXAMPLE 15

### Compounds AA, AB and AC

A stirred suspension of 4,5-diphenylimidazole-2-thiol (5.04g) in dry tetrahydrofuran (150ml) was treated with sodium hydride (60% dispersion in oil;0.88g;22mmol) at the ambient temperature under an inert atmosphere, and stirred for 30 minutes. The resulting solution was treated with 1,2-bis(bromomethyl)benzene (2.64g) and stirred for a further period of 1 hour. The mixture was then heated at reflux for 30 minutes and then it was poured into brine (200ml). The organic layer was dried over magnesium sulphate and evaporated and the resulting residue was extracted with boiling 2-ethoxyethanol (1x500ml). Concentration of the extract (to about 50ml) gave a solid which was filtered off, washed with diethyl ether and dried, to give 1,2-bis(4,5-diphenylimidazol-2-ylthiomethyl)benzene (1.75g), in the form of a colourless solid, m.p. 255-257°C. [Elemental analysis:- C,74.90; H,5.01;N,9.20;S,10.80%; calculated:- C,75.21;H,4.98; N,9.23;S,10.57%].

By proceeding in a similar manner, but replacing the 1,2-bis(bromomethyl)benzene by the appropriate quantities of 1,3-bis(bromomethyl)benzene and 1,4-bis(bromomethyl)benzene, respectively, there were prepared:-
1,3-bis(4,5-diphenylimidazol-2-ylthiomethyl)benzene in the form of a colourless solid, m.p. 231-232°C [Elemental analysis:- C,75.60;H,5.17;N,8.90;S,10.70%; calculated:- C,75.21;H,4.98;N,9.23;S,10.57%]; and 1,4-bis(4,5-diphenylimidazol-2-ylthiomethyl)benzene in the form of a colourless solid, m.p. 259-260°C [Elemental analysis:- C,74.80;H,4.98;N,9.10;S,10.40%; calculated:- C,75.21;H,4.98;N,9.23;S,10.57%].

### EXAMPLE 16

### Compounds AD and AE

A stirred suspension of 4,5-diphenylimidazole-2-thiol (10.0g) and 1-methyl-2-mercaptoimidazole (4.54g) in dry tetrahydrofuran (300ml) was treated with sodium hydride (60% dispersion in oil;2.66g) under an inert atmosphere at ambient temperature, and stirred for 20 minutes. The resulting solution was treated with 1,3-dichloropropan-2-ol (3.8ml) and the mixture was stirred at the ambient temperature for 20 hours. The mixture was then evaporated and the residue was partitioned between water (500ml) and ethyl acetate (500ml). The ethyl acetate solution was dried over magnesium sulphate, and evaporated and the resulting solid residue was subjected to flash chromatography on silica gel, using ethyl acetate as eluent.

The first major component obtained was 1,3-bis(4,5-diphenylimidazol-2-ylthio)propan-2-ol (1.8g) in the form of a colourless solid, m.p. 225-227°C [Elemental analysis:- C,70.30;H,5.10;N,9.70; S,11.40%; calculated:- C,70.68;H,5.03;N,9.99;S 11.43%]; and the second major component obtained was 4,5-diphenyl-2-[3-(1-methylimidazol-2-ylthio)-2-hydroxypropylthio]imidazole (3.5g) in the form of a colourless solid, m.p. 198-200°C [Elemental analysis:-C,62.40;H,5.23;N,13.20;S,15.30%; calculated:- C,62.53; H,5.24;N,13.26;S,15.17%].

### EXAMPLE 17

### Compound BA

A solution of maleic acid (30.47g) in acetone (200ml) was added to a stirred suspension of 4,5-diphenyl-2-[3-(1-methylimidazol-2-ylthio)propylthio]imidazole (50.82g) in acetone (400ml). The suspension was stirred at ambient temperature; a clear solution formed, then a solid crystallised. The solid was collected, was washed with diethyl ether and was then dried to give 4,5-diphenyl-2-[3-(1-methylimidazol-2-ylthio)propylthio]imidazole dimaleate in the form of a white powder; m.p. 92-94°C. [Elemental analysis:-C,55.0;H,4.7;N,8.6;S,9.7%; calculated for C₃₀H₃₀N₄O₈S₂:H₂O:- C,54.87;H,4.91;N,8.53;S,9.76%].

### EXAMPLE 18

### Compounds BB to CC

By carrying out processes similar to those described herein, more especially in the Examples and Reference Examples, there were prepared the following compounds:-
4,5-Diphenyl-2-[3-(3-amino-1,2,4-triazol-5-ylthio)propylthio]imidazole, m.p. 171-173°C from ethanol; 4,5-Diphenyl-2-[3-(pyrid-2-ylthio)propylthio]imidazole, m.p. 120-122°C after chromatography on silica eluting with a mixture of dichloromethane and ethyl acetate;
E-4,5-Diphenyl-2-[4-(1-methylimidazol-2-ylthio)but-2-enylthio]imidazole, m.p. 125-127°C from t-butyl methyl ether;
4,5-Diphenyl-2-[3-(pyrimidin-2-ylsulphonyl)propylthio]imidazole, m.p. 144-145°C from ethyl acetate;
4,5-Diphenyl-2-[4-(pyrimidin-2-ylsulphonyl)butylthio]imidazole, m.p. 138-139°C from ethyl acetate;
4,5-Diphenyl-2-[3-(pyrid-2-ylsulphinyl)propylthio]imidazole, m.p. 108-109°C from ethyl acetate;
4,5-Diphenyl-2-[4-(pyrimidin-2-ylthio)butylthio]imidazole, m.p. 110°C from diethyl ether;
1,6-Bis(4,5-diphenylimidazol-2-ylthio)hexane, m.p. 205-207°C from acetonitrile;
4,5-Bis(4-methylphenyl)-2-[3-(1-methylimidazol-2-ylthio)propylthio]imidazole, m.p. 141-142°C from a mixture of ethyl acetate and cyclohexane;
4,5-Bis(4-methoxyphenyl)-2-[3-(pyrimidin-2-ylsulphonyl)propylthio)imidazole, m.p. 57-63°C after trituration with light petroleum (b.p. 60 - 80°C.);
4,5-Diphenyl-2-[4-(imidazol-2-ylthio)butylthio]imidazole, m.p. 155-156°C from ethyl acetate;
4,5-Diphenyl-2-[3-(pyrid-4-ylsulphonyl)propylthio]imidazole, m.p. 171-173°C from ethyl acetate;
4,5-Diphenyl-2-[3-(pyrid-2-ylsulphonyl)propylthio)imidazole, m.p. 143-144°C after trituration with diethyl ether;
4,5-Diphenyl-2-[4-(pyrid-2-ylthio)butylthio]imidazole, m.p. 122-124°C from a mixture of toluene and light petroleum;
1,5-Bis(4,5-diphenylimidazol-2-ylthio)pentane, m.p. 211-213°C from ethyl acetate;
1,4-Bis(4,5-diphenylimidazol-2-ylthio)but-2-yne, m.p. 200-202°C from ethyl acetate;
4,5-Diphenyl-2-[4-(1-methylimidazol-2-ylthio)but-2-ynylthio]imidazole, m.p. 150-152°C from isopropanol;
4,5-Bis(4-fluorophenyl)-2-[3-(1-methylimidazol-2-ylthio)propylthio]imidazole, m.p. 178-180°C from a mixture of ethyl acetate and cyclohexane;
4,5-Bis(4-methoxyphenyl)-2-[4-(pyrimidin-2-ylsulphonyl)butylthio]imidazole, m.p. 82-84°C after trituration with light petroleum;
4,5-Diphenyl-2-[4-(pyrid-2-ylsulphonyl)butylthio]imidazole, m.p. 107-108°C from ethyl acetate;
4,5-Diphenyl-2-[4-(pyrid-2-ylsulphinyl)butylthio]imidazole, m.p. 82-84°C after trituration with diisopropyl ether;
4,5-Diphenyl-2-{3-[4(5)-methylimidazol-2-ylthio]propylthio}imidazole, m.p. 185°C from a mixture of ethyl acetate and light petroleum;
4,5-Diphenyl-2-[5-(imidazol-2-ylthio)pentylthio]imidazole, m.p. 165-166°C from a mixture of ethanol and ethyl acetate;
4,5-Diphenyl-2-[3-(imidazol-2-ylthio)propylthio]imidazole dihydrochloride, m.p. 208-210°C after trituration with diethyl ether [Elemental analysis:- C,53.8;H,4.7; N,11.9;Cl,15.68;S,13.6%; calculated for C₂₁H₂₀N₄S₂:2HCl:- C,54.17;H,4.76;N,12.0;Cl,15.23; S,13.77%];
4,5-Diphenyl-2-[3-(imidazol-2-ylthio)propylthio]imidazole dimaleate, m.p. 162-164°C from water [Elemental analysis:- C,54.6;H,4.5;N,8.9;S,10.2%; calculated for C₂₉H₂₈N₄O₈S₂:0.5H₂O:- C,54.97;H,4.61;N,8.84;S,10.12%]; Z-1,4-Bis(4,5-diphenylimidazol-2-ylthio)but-2-ene, m.p. 215-217°C from isopropanol, [Elemental analysis:-C,72.1;H,5.14;N,9.7;S,11.3;water,0.9%; calculated for C₃₄H₂₈N₄S₂:0.5H₂O:- C,72.18;H,5.17;N,9.90;S,11.33; water,1.59%];
4,5-Diphenyl-2-[4-(pyrid-2-ylsulphonyl)butylthio]imidazole hydrochloride, m.p. 156-158°C after trituration with diethyl ether [Elemental analysis:- C,58.8; H,5.03;N,8.2;Cl,7.2;S,13.0;water,1.7%; calculated for C₂₄H₂₃N₃S₂O₂:HCl:0.4H₂O:- C,58.49;H,5.23;N,8.52; Cl,7.42;S,13.01;water,1.46%]; and
4,5-Diphenyl-2-[4-(imidazol-2-ylsulphonyl)butylthio]imidazole, m.p. 215°C from ethanol [Elemental analysis:-C,59.1;H,5.0;N,12.6;S,14.5%; calculated for C₂₂H₂₂N₄S₂O₂:H₂O:- C,59.3;H,4.94;N,12.58;S,14.4%].

### REFERENCE EXAMPLE 1

Sodium hydride (1.6g; 80% dispersion in mineral oil) was carefully added to a stirred solution of 1-methylimidazole-2-thiol (5.7g) in dry tetrahydrofuran (100ml). After 15 minutes, 1-chloro-3-iodopropane (10.2g) was added and the mixture was stirred overnight at ambient temperature. The mixture was concentrated under reduced pressure and the residue was partitioned between water(50ml) and ethyl acetate (75ml). The organic solution was washed with water, dried over magnesium sulphate and concentrated under reduced pressure to yield 1-methyl-2-(3-chloropropylthio)imidazole as a yellow oil which was utilised without further purification.

A solution of 1-methyl-2-(3-chloropropylthio)imidazole (8.8g) in dry dichloromethane (100ml) was cooled to 10°C and m-chloroperbenzoic acid (27.6g; 72% mixture with water) was added. The mixture was stirred for 12 hours at ambient temperature and then filtered. The filtrate was washed twice with 10% aqueous sodium metabisulphite, then with 10% aqueous sodium bicarbonate and finally twice with water, then dried over magnesium sulphate. Concentration under reduced pressure left a yellow oil that crystallised on standing. Chromatography of the solid on silica eluting with diethyl ether provided 1-methyl-2-(3-chloropropylsulphonyl)imidazole as a yellow oil.

### REFERENCE EXAMPLE 2

Potassium t-butoxide (12.3g) was added to 4,5-diphenyl-2-mercaptoimidazole (25.2g) in dry DMF (300ml) and the mixture was stirred it ambient temperature for 30 minutes, then 1-chloro-3-iodopropane (21.0g) was added and the mixture was stirred overnight. The residue left after concentrating under reduced pressure was partitioned between ethyl acetate and water. The aqueous fraction was twice extracted with ethyl acetate and then the combined ethyl acetate solutions were washed with water and dried over magnesium sulphate. Chromatography on silica, eluting with chloroform provided 2-(3-chloropropylthio)-4,5-diphenylimidazole, in the form of a white solid from ethyl acetate / petrol, m.p. 147-150°C; [Found:-C,65.6;H,5.21;N,8.4;Cl,10.9;S,9.8%; Calculated for C₁₈H₁₇ClN₂S:- C,65.74;H,5.21;N,8.52;Cl,10.78;S,9.75%].

In clinical practice compounds of the present invention may be administered parenterally, rectally or orally.

Solid compositions for oral administration include compressed tablets, pills, powders and granules. In such solid compositions, one or more of the active compounds is, or are, admixed with at least one inert diluent such as starch, sucrose or lactose. The compositions may also comprise, is is normal practice, additional substances other than inert diluents, e.g. lubricating agents, such as magnesium stearate.

Liquid compositions for oral administration include pharmaceutically acceptable emulsions, solutions, suspensions, syrups and elixirs containing inert diluents commonly used in the art such as water and liquid paraffin. Besides inert diluents such compositions may comprise adjuvants, such as wetting and suspending agents, and sweetening, flavouring, perfuming and preserving agents. The compositions according to the invention for oral administration also include capsules of absorbable material such as gelatin, containing one or more of the active substances with or without the addition of diluents or excipients.

Compositions according to the invention for parenteral administration include sterile aqueous, aqueous-organic, and organic solutions, suspensions and emulsions. Examples of organic solvents or suspending media are propylene glycol, polyethylene glycol, vegetable oils such as olive oil and injectable organic esters such as ethyl oleate. The compositions may also contain adjuvants such as stabilising, preserving, wetting, emulsifying and dispersing agents. They may be sterilised, for example, by filtration through a bacteria-retaining filter, by incorporation in the compositions of sterilising agents, by irradiation or by heating. They may also be manufactured in the form of sterile solid compositions, which can be dissolved in sterile water or some other sterile injectable medium immediately before use.

Solid compositions for rectal administration include suppositories formulated in accordance with known methods and containing at least one compound of formula (I).

The percentage of active ingredient in the compositions of the invention may be varied, it being necessary that it should constitute a proportion such that a suitable dosage shall be obtained. Obviously, several unit dosage forms may be administered at about the same time. The dose employed will be determined by the physician, and depends upon the desired therapeutic effect, the route of administration and the duration of the treatment, and the condition of the patient. In the adult, the doses are generally from 0.5 to 70, preferably 1 to 10, mg/kg body weight per day by oral administration.

The following Examples illustrate pharmaceutical compositions according to the present invention.

### COMPOSITION EXAMPLE 1

No. 2 size gelatin capsules each containing:-

| | |
|---|---|
| 2-[3-(1-methylimidazol-2-ylthio)propylthio]-4,5-diphenylimidazole | 20 mg |
| lactose | 100 mg |
| starch | 60 mg |
| dextrin | 40 mg |
| magnesium stearate | 1 mg |

were prepared in accordance with the usual procedure.

### COMPOSITION EXAMPLE 2

No. 2 size gelatin capsules each containing:-

| | |
|---|---|
| 1,2-bis(4,5-diphenylimidazol-2-ylthiomethyl)benzene | 20 mg |
| lactose | 100 mg |
| starch | 60 mg |
| dextrin | 40 mg |
| magnesium stearate | 1 mg |

were prepared in accordance with the usual procedure.

### COMPOSITION EXAMPLE 3

No. 2 size gelatin capsules each containing:-

| | |
|---|---|
| 2-[3-(imidazol-2-ylthio)propylthio]-4,5-diphenylimidazole | 20 mg |
| lactose | 100 mg |
| starch | 60 mg |
| dextrin | 40 mg |
| magnesium stearate | 1 mg |

were prepared in accordance with the usual procedure.

## Claims

1. An imidazole derivative of the general formula: 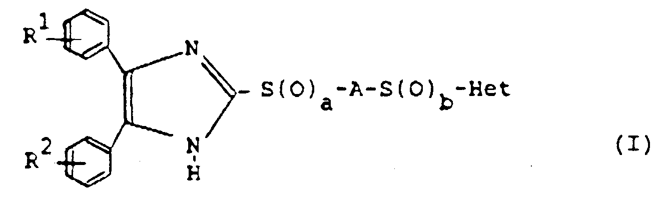 wherein:
A represents a methylene group or a group -CH₂-A'-CH₂- wherein A' represents a direct bond, a linear alkanediyl, alkenediyl or alkynediyl group containing up to 14 carbon atoms, or a hydroxymethylene group, or A' represents a phenylene group optionally substituted by 1 to 4 substituents selected from halogen atoms, alkyl groups (optionally substituted by one or more halogen atoms), alkoxy, alkylthio, alkylamino, carboxy and alkoxycarbonyl groups;
R¹ and R² are the same or different and each represents a hydrogen or halogen atom, an alkyl group (optionally substituted by one or more halogen atoms), or an alkoxy, alkylthio, alkylamino, carboxy or alkoxycarbonyl group;
a and b are the same or different and each represents 0, 1 or 2; and
Het represents a heterocyclic group containing from 5 to 7 ring atoms chosen from carbon, nitrogen, sulphur and oxygen atoms, optionally substituted by one or two substituents independently selected from:-
halogen atoms, alkyl groups (optionally substituted by one or more halogen atoms), hydroxy, amino, alkoxy, alkylthio, alkylamino, carboxy, alkoxycarbonyl and pyridyl groups, and phenyl groups (optionally substituted by one or more substituents selected from halogen atoms, alkyl groups (optionally substituted by one or more halogen atoms), and alkoxy, alkylthio, alkylamino, carboxy and alkoxycarbonyl groups];
and pharmaceutically acceptable salts thereof; wherein all alkyl groups and moieties, unless otherwise indicated, are straight- or branched-chain and contain from 1 to 5 carbon atoms; and halogen atoms, unless otherwise indicated, are fluorine, chlorine, bromine or iodine atoms; for use in the preparation of a pharmaceutical composition for the treatment of a condition, which can be ameliorated by administration of an inhibitor of acyl coenzyme-A: cholesterol-O-acyl transferase.

2. A compound according to claim 1 for use in the preparation of a pharmaceutical composition for the treatment of atherosclerosis, hyperlipidaemia, cholesterol ester storage disease or atheroma in vein grafts.

3. An imidazole derivative of general formula I, as defined in claim 1, or a pharmaceutically acceptable salt thereof, provided that when R¹ and R² are identical and each represents a fluorine, chlorine or bromine atom or a trifluoromethyl, methoxy or methylthio group in the para position and A represents a methylene, 1,2-ethanediyl, 1,3-propanediyl, 2-hydroxy-1,3-propanediyl or 1,4-butanediyl group then Het represents other than a group of general formula: 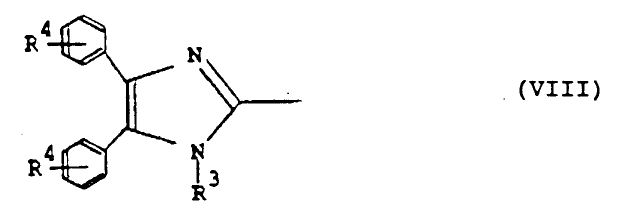 wherein R³ represents a hydrogen atom or a methyl group and the symbols R⁴ are identical and each represents a fluorine, chlorine or bromine atom or a trifluoromethyl, methoxy or methylthio group in the para position, for use in a method of treatment of the human or animal body by therapy.

4. A compound according to claim 3 for use in a method of treatment of the human or animal body by therapy of atherosclerosis, hyperlipidaemia, cholesterol ester storage disease or atheroma in vein grafts.

5. An imidazole derivative of general formula I, as defined in claim 1 or a pharmaceutically acceptable salt thereof, provided that:-
(1) when R¹ and R² are identical and each represents a fluorine, chlorine or bromine atom or a trifluoromethyl, methoxy or methylthio group in the para position and A represents a methylene, 1,2-ethanediyl, 1,3-propanediyl, 2-hydroxy-1,3-propanediyl, or 1,4-butanediyl group, then Het represents other than a group of formula (VIII), wherein R³ represents a hydrogen atom or a methyl group and the symbols R⁴ are identical and each represents a fluorine, chlorine or bromine atom or a trifluoromethyl, methoxy or methylthio group in the para position; and
(2) when A represents a 1,3-propanediyl or 2-hydroxy-1,3-propanediyl group, R¹ and R² both represent hydrogen atoms, and a and b both represent zero, then Het represents other than a group of formula (VIII) wherein R³ represents a hydrogen atom and the symbols R⁴ both represent hydrogen atoms.

6. A compound according to claim 5 wherein at least one of the symbols has a value selected from the following:-
(i) A represents a group -CH₂-A'-CH₂- wherein A' represents a direct bond, a linear alkanediyl, alkenediyl or alkynediyl group containing up to 7 carbon atoms or a hydroxymethylene group, or a substituted or unsubstituted phenylene group;
(ii) R¹ and R² are the same or different and each represents a hydrogen or halogen atom, or an alkyl or alkoxy group; and
(iii) Het represents an optionally substituted 5- or 6-membered heterocyclic group containing up to 4 nitrogen atoms, unsubstituted or substituted by alkyl, amino, pyridyl or optionally substituted phenyl.

7. A compound according to claim 5 or 6 wherein at least one of the symbols has a value selected from the following:
(i) A' represents a methylene group or a linear alkanediyl group containing 2, 3, 4 or 7 carbon atoms or a vinylene or ethynylene group or an unsubstituted phenylene group;
(ii) R¹ and R² are the same or different and each represents a hydrogen atom or a fluorine atom or a methyl or methoxy group in the 4-position; and
(iii) Het represents a pyrimidin-2-yl, pyrid-2-yl, pyrid-4-yl, imidazol-2-yl, 1,2,4-triazol-3-yl, or tetrazol-5-yl, group unsubstituted or substituted by methyl, amino, pyrid-2-yl or optionally substituted phenyl.

8. A compound according to claim 1 which is:
A 2-[3-(Pyrimidin-2-ylthio)propylthio]-4,5-diphenylimidazole
B 2-[3-(Pyrid-4-ylthio)propylthio]-4,5-diphenylimidazole
C 2-[3-(1-Methylimidazol-2-ylthio)propylthio]-4,5-diphenylimidazole
D 2-[3-(1-Methylimidazol-2-ylthio)propylthio]-4,5-diphenylimidazole dihydrochloride
E 1,2-Bis(4,5-diphenylimidazol-2-ylthio)ethane
G 2-[3-(4,6-Dimethylpyrimidin-2-ylthio)propylthio]-4,5-diphenylimidazole
H 2-[3-(1-Methylimidazol-2-ylsulphonyl)propylthio]-4,5-diphenylimidazole
I 2-[3-(4-Methyl-1,2,4-triazol-3-ylthio)propylthio]-4,5-diphenylimidazole
J 2-[3-(1-Methyltetrazol-5-ylthio)propylthio]-4,5-diphenylimidazole
K 1-(4,5-Diphenylimidazol-2-ylthio)-3-[(4,5-dipyrid-2-yl)imidazol-2-ylthio]propane dihydrochloride
L 2-[3-(1-Methyltetrazol-5-ylsulphonyl)propylthio]-4,5-diphenylimidazole
M 2-[3-(Imidazol-2-ylthio)propylthio]-4,5-diphenylimidazole
N 2-[4-(1-Methylimidazol-2-ylthio)butylthio]-4,5-diphenylimidazole
O 2-[4-(1-Methylimidazol-2-ylsulphonyl)butylsulphonyl]-4,5-diphenylimidizole
P 1,4-Bis(4,5-diphenylimidazol-2-ylthio)butane
Q E-1,4-Bis(4,5-diphenylimidazol-2-ylthio)but-2-ene
R 2-[6-(1-Methylimidazol-2-ylthio)hexylthio]-4,5-diphenylimidazole
S 1,9-Bis(4,5-diphenylimidazol-2-ylthio)nonane
T 2-[9-(1-Methylimidazol-2-ylthio)nonylthio]-4,5-diphenylimidazole
AA 1,2-bis(4,5-diphenylimidazol-2-ylthiomethyl)benzene
AB 1,3-bis(4,5-diphenylimidazol-2-ylthiomethyl)benzene
AC 1,4-bis(4,5-diphenylimidazol-2-ylthiomethyl)benzene
AE 4,5-diphenyl-2-[3-(1-methylimidazol-2-ylthio)-2-hydroxypropylthio]imidazole
BA 4,5-diphenyl-2-[3-(1-methylimidazol-2-ylthio)propylthio]imidazole dimaleate
BB 4,5-Diphenyl-2-[3-(3-amino-1,2,4-triazol-5-ylthio)propylthio]imidazole
BC 4,5-Diphenyl-2-[3-(pyrid-2-ylthio)propylthio]imidazole
BD E-4,5-Diphenyl-2-[4-(1-methylimidazol-2-ylthio)but-2-enylthio]imidazole
BE 4,5-Diphenyl-2-[3-(pyrimidin-2-ylsulphonyl)propylthio]imidizole
BF 4,5-Diphenyl-2-[4-(pyrimidin-2-ylsulphonyl)butylthio]imidazole
BG 4,5-Diphenyl-2-[3-(pyrid-2-ylsulphinyl)propylthio]imidazole
BH 4,5-Diphenyl-2-[4-(pyrimidin-2-ylthio)butylthio]imidazole
BI 1,6-Bis(4,5-diphenylimidazol-2-ylthio)hexane
BJ 4,5-Bis(4-methylphenyl)-2-[3-(1-methylimidizol-2-ylthio)propylthio]imidizole
BK 4,5-Bis(4-methoxyphenyl)-2-[3-(pyrimidin-2-ylsulphonyl)propylthio]imidazole
BL 4,5-Diphenyl-2-[4-(imidazol-2-ylthio)butylthio]imidazole
BM 4,5-Diphenyl-2-[3-(pyrid-4-ylsulphonyl)propylthio]imidazole
BN 4,5-Diphenyl-2-[3-(pyrid-2-ylsulphonyl)propylthio]imidazole
BO 4,5-Diphenyl-2-[4-(pyrid-2-ylthio)butylthio]imidazole
BP 1,5-Bis(4,5-diphenylimidazol-2-ylthio)pentane
BQ 1,4-Bis(4,5-diphenylimidazol-2-ylthio)but-2-yne
BR 4,5-Diphenyl-2-[4-(1-methylimidazol-2-ylthio)but-2-ynylthio]imidazole
BS 4,5-Bis(4-fluorophenyl)-2-[3-(1-methylimidazol-2-ylthio)propylthio]imidazole
BT 4,5-Bis(4-methoxyphenyl)-2-[4-(pyrimidin-2-ylsulphonyl)butylthio]imidazole
BU 4,5-Diphenyl-2-[4-(pyrid-2-ylsulphonyl)butylthio]imidazole
BV 4,5-Diphenyl-2-[4-(pyrid-2-ylsulphinyl)butylthio]imidazole
BW 4,5-Diphenyl-2-{3-[4(5)-methylimidazol-2-ylthio]propylthio)imidazole
BX 4,5-Diphenyl-2-[5-(imidazol-2-ylthio)pentylthio]imidazole
BY 4,5-Diphenyl-2-[3-(imidazol-2-ylthio)propylthio]imidazole dihydrochloride
BZ 4,5-Diphenyl-2-[3-(imidazol-2-ylthio)propylthio]imidazole dimaleate
CA Z-1,4-Bis(4,5-diphenylimidazol-2-ylthio)but-2-ene
CB 4,5-Diphenyl-2-[4-(pyrid-2-ylsulphonyl)butylthio]imidazole hydrochloride
CC 4,5-Diphenyl-2-[4-(imidazol-2-ylsulphonyl)butylthio]imidazole.

9. A process for the preparation of a compound of general formula (I) as defined in claim 5 which comprises:
(A) when A, R¹, R², b and Het are as defined in claim 5 and a represents zero, the reaction of a salt of general formula: 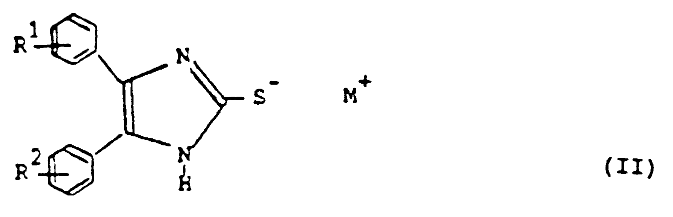 wherein R¹ and R² are as defined in claim 5 and M represents an alkali metal atom, with a compound of formula:
Z¹-A-S(O)_{b}-Het (III)
wherein A, b and Het are as defined in claim 5 and Z¹ represents a chlorine, bromine or iodine atom, or an alkyl- or aryl-sulphonyloxy group;
(B) when A, R¹, R², a and Het are as defined in claim 5 and b represents zero, the reaction of a salt of formula:
Het-S⁻ M⁺ (V)
wherein Het and M are as hereinbefore defined, with a compound of general formula: 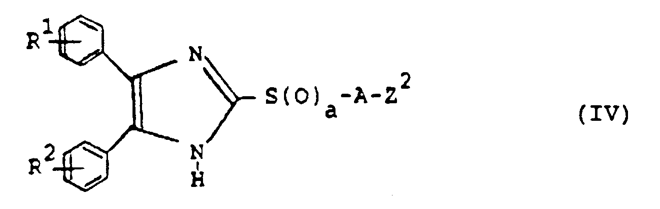 wherein A, R¹, R² and a are as defined in claim 5 and Z² represents a chlorine, bromine or iodine atom, or an alkyl- or aryl-sulphonyloxy group;
(C) when the compound of formula (I) conforms to the general formula: 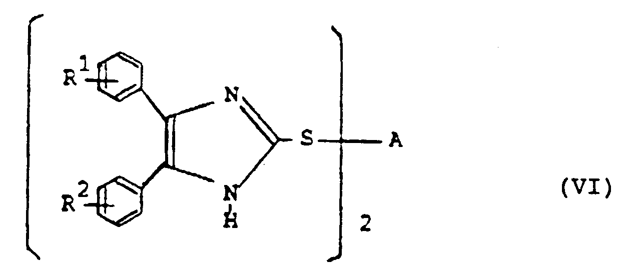 wherein A, R¹ and R² are as defined in claim 5, the reaction of a salt of formula (II) with a compound of formula:
Z¹-A-Z² (VII)
wherein A, Z¹ and Z² are as hereinbefore defined;
(D) when A; R¹, R² and Het are as defined in claim 5 and a and b are both zero, the reaction of a salt of formula (II) and a salt of formula (V) with a compound of formula (VII) wherein the various symbols are as hereinbefore defined;
(E) when A, R¹, R² and Het are as hereinbefore defined and at least one of a and b represents 1 or 2, the oxidation of a compound of formula (I), wherein A, R¹ and Het are as hereinbefore defined and a and/or b is less than in the desired product;
optionally followed by the conversion of a compound of general formula (I) into another compound of general formula (I);
optionally followed by conversion of a compound of general formula (I) into a pharmaceutically acceptable salt thereof.

10. A pharmaceutical composition which comprises an imidazole derivative of formula (I) as defined in claim 3, or a pharmaceutically acceptable salt thereof, in association with a pharmaceutically acceptable carrier or coating.

## Patentansprüche

1. Imidazolderivat der allgemeinen Formel: 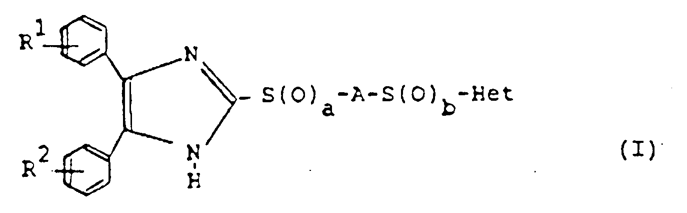 worin bedeuten:
A eine Methylengruppe oder eine Gruppe -CH₂-A'-CH₂- mit A' gleich einer direkten Bindung, einer linearen Alkandiyl-, Alkendiyl- oder Alkindiylgruppe mit bis zu 14 Kohlenstoffatomen oder einer Hydroxymethylengruppe oder A' gleich einer gegebenenfalls ein- bis vierfach durch Halogenatome, Alkylgruppen (gegebenenfalls substituiert durch ein oder mehrere Halogenatome), Alkoxy-, Alkylthio-, Alkylamino-, Carboxy- und Alkoxycarbonylgruppen substituierten Phenylengruppe;
R¹ und R², die gleich oder verschieden sein können, jeweils ein Wasserstoff- oder Halogenatom, eine Alkylgruppe (gegebenenfalls substituiert durch ein oder mehrere Halogenatome) oder eine Alkoxy-, Alkylthio-, Alkylamino-, Carboxy- oder Alkoxycarbonylgruppe;
a und b, die gleich oder verschieden sein können, jeweils 0, 1 oder 2 und
Het eine 5 bis 7 Ringatome, die unter Kohlenstoff-, Stickstoff-, Schwefel- und Sauerstoffatomen ausgewählt sind, enthaltende heterocyclische Gruppe, die gegebenenfalls durch einen oder zwei Substituenten substituiert ist, wobei die Substituenten unabhängig voneinander unter Halogenatomen, Alkylgruppen (gegebenenfalls substituiert durch ein oder mehrere Halogenatome), Hydroxy-, Amino-, Alkoxy-, Alkylthio-, Alkylamino-, Carboxy-, Alkoxycarbonyl- und Pyridylgruppen sowie Phenylgruppen [gegebenenfalls substituiert durch einen oder mehrere aus Halogenatomen, Alkylgruppen (gegebenenfalls substituiert durch ein oder mehrere Halogenatome) und Alkoxy-, Alkylthio-, Alkylamino-, Carboxy- und Alkoxycarbonylgruppen ausgewählte(n) Substituenten] ausgewählt sind,
und pharmazeutisch akzeptable Salze hiervon, wobei alle Alkylgruppen und -einheiten, sofern nicht anders angegeben, gerad- oder verzweigtkettig sind und ein bis fünf Kohlenstoffatom(e) aufweisen und Halogenatome, sofern nicht anders angegeben, Fluor-, Chlor-, Brom- oder Jodatome sind,
zur Verwendung bei der Herstellung einer Arzneimittelzubereitung zur Behandlung eines Zustands, der durch Verabreichung eines Hemmers von Acyl-Coenzym A: Cholesterin-O-Acyltransferase verbessert werden kann.

2. Verbindung nach Anspruch 1 zur Verwendung bei der Herstellung einer Arzneimittelzubereitung zur Behandlung von Atherosklerose, Hyperlipidämie, einer Cholesterinesterspeicherkrankheit oder eines Ateroms bei Gefäßimplantaten.

3. Imidazolderivat der allgemeinen Formel I gemäß der Definition in Anspruch 1 oder ein pharmazeutisch akzeptables Salz hiervon, vorausgesetzt, daß, wenn R¹ und R² identisch sind und jeweils für ein Fluor-, Chlor- oder Bromatom oder eine Trifluormethyl-, Methoxy- oder Methylthiogruppe in para-Stellung stehen und A eine Methylen-, 1,2-Ethandiyl-, 1,3-Propandiyl-, 2-Hydroxy-1,3-propandiyl- oder 1,4-Butandiylgruppe bedeutet, Het eine Gruppe darstellt, die sich von einer Gruppe der folgenden allgemeinen Formel unterscheidet: 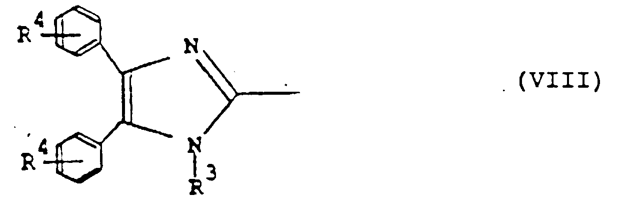 worin R³ für ein Wasserstoffatom oder eine Methylgruppe steht und die Symbole R⁴ identisch sind und jeweils für ein Fluor-, Chlor- oder Bromatom oder eine Trifluormethyl-, Methoxy- oder Methylthiogruppe in para-Position stehen,
zur Verwendung bei einem Verfahren zur therapeutischen Behandlung des menschlichen oder tierischen Körpers.

4. Verbindung nach Anspruch 3 zur Verwendung bei einem Verfahren zur therapeutischen Behandlung von Atherosklerose, Hyperlipidämie, Cholesterinesterspeichererkrankung oder Aterom bei Gefäßplastiken am menschlichen oder tierischen Körper.

5. Imidazolderivat der allgemeinen Formel I gemäß der Definition in Anspruch 1 oder ein pharmazeutisch akzeptables Salz hiervon, wobei gilt:
1) wenn R¹ und R² identisch sind und jeweils für ein Fluor-, Chlor- oder Bromatom oder eine Trifluormethyl-, Methoxy- oder Methylthiogruppe in para-Position stehen und A eine Methylen-, 1,2-Ethandiyl-, 1,3-Propandiyl-, 2-Hydroxy-1,3-propandiyl- oder 1,4-Butandiylgruppe bedeutet, stellt Het eine Gruppe dar, die von einer Gruppe der Formel VIII verschieden ist, worin R³ für ein Wasserstoffatom oder eine Methylgruppe steht und die Symbole R⁴ identisch sind und jeweils ein Fluor-, Chlor- oder Bromatom oder eine Trifluormethyl-, Methoxy- oder Methylthiogruppe in para-Position bedeuten, und
2) wenn A für eine 1,3-Propandiyl- oder 2-Hydroxy-1,3-propandiylgruppe steht, R¹ und R² beide Wasserstoffatome bedeuten und a und b beide 0 sind, stellt Het eine Gruppe dar, die von einer Gruppe der Formel VIII verschieden ist, worin R³ für ein Wasserstoffatom steht und die beiden Symbole R⁴ Wasserstoffatome bedeuten.

6. Verbindung nach Anspruch 5, worin mindestens eines der Symbole eine Bedeutung hat, die aus den folgenden ausgewählt ist:
i) A bedeutet eine Gruppe -CH₂-A'-CH₂- mit A' gleich einer direkten Bindung, einer linearen Alkandiyl-, Alkendiyl- oder Alkindiylgruppe mit bis zu 7 Kohlenstoffatomen oder einer Hydroxymethylengruppe oder einer substituierten oder unsubstituierten Phenylengruppe;
ii) R¹ und R², die gleich oder verschieden sein können, bedeuten jeweils ein Wasserstoff- oder Halogenatom oder eine Alkyl- oder Alkoxygruppe und
iii) Het bedeutet eine gegebenenfalls substituierte 5- oder 6-gliedrige heterocyclische Gruppe mit bis zu 4 Stickstoffatomen, die nicht substituiert oder durch Alkyl, Amino, Pyridyl oder gegebenenfalls substituiertes Phenyl substituiert ist.

7. Verbindung nach Anspruch 5 oder 6, wobei mindestens eines der Symbole eine Bedeutung aufweist, die aus den folgenden ausgewählt ist:
i) A' bedeutet eine Methylengruppe oder eine lineare Alkandiylgruppe mit 2, 3, 4 oder 7 Kohlenstoffatomen oder eine Vinylen- oder Ethinylengruppe oder eine unsubstituierte Phenylengruppe;
ii) R¹ und R², die gleich oder verschieden sein können, bedeuten jeweils ein Wasserstoffatom oder ein Fluoratom oder eine Methyl- oder Methoxygruppe in 4-Position und
iii) Het bedeutet eine Pyrimidin-2-yl-, Pyrid-2-yl-, Pyrid-4-yl-, Imidazol-2-yl-, 1,2,4-Triazol-3-yl- oder Tetrazol-5-yl-Gruppe, die nicht substituiert oder durch Methyl, Amino, Pyrid-2-yl oder gegebenenfalls substituiertes Phenyl substituiert ist.

8. Verbindung nach Anspruch 1, nämlich:
A 2-[3-(Pyrimidin-2-ylthio)propylthio]-4,5-diphenylimidazol
B 2-[3-(Pyrid-4-ylthio)propylthio]-4,5-diphenylimidazol
C 2-[3-(1-Methylimidazol-2-ylthio)propylthio]-4,5-diphenylimidazol
D 2-[3-(1-Methylimidazol-2-ylthio)propylthio]-4,5-diphenylimidazol-dihydrochlorid
E 1,2-Bis(4,5-diphenylimidazol-2-ylthio)ethan
G 2-[3-(4,6-Dimethylpyrimidin-2-ylthio)propylthio]-4,5-diphenylimidazol
H 2-[3-(1-Methylimidazol-2-ylsulfonyl)propylthio]-4,5-diphenylimidazol
I 2-[3-(4-Methyl-1,2,4-triazol-3-ylthio)propylthio]-4,5-diphenylimidazol
J 2-[3-(1-Methyltetrazol-5-ylthio)propylthio]-4,5-diphenylimidazol
K 1-(4,5-Diphenylimidazol-2-ylthio)-3-[(4,5-dipyrid-2-yl)imidazol-2-ylthio]propan-dihydrochlorid
L 2-[3-(1-Methyltetrazol-5-ylsulfonyl)propylthio]-4,5-diphenylimidazol
M 2-[3-(Imidazol-2-ylthio)propylthio]-4,5-diphenylimidazol
N 2-[4-(1-Methylimidazol-2-ylthio)butylthio]-4,5-diphenylimidazol
O 2-[4-(1-Methylimidazol-2-ylsulfonyl)butylsulfonyl]-4,5-diphenylimidazol
P 1,4-Bis(4,5-diphenylimidazol-2-ylthio)butan
Q E-1,4-Bis(4,5-diphenylimidazol-2-ylthio)-but-2-en
R 2-[6-(1-Methylimidazol-2-ylthio)hexylthio]-4,5-diphenylimidazol
S 1,9-Bis(4,5-diphenylimidazol-2-ylthio)nonan
T 2-[9-(1-Methylimidazol-2-ylthio)nonylthio]-4,5-diphenylimidazol
AA 1,2-Bis(4,5-diphenylimidazol-2-ylthiomethyl)benzol
AB 1,3-Bis(4,5-diphenylimidazol-2-ylthiomethyl)benzol
AC 1,4-Bis(4,5-diphenylimidazol-2-ylthiomethyl)benzol
AE 4,5-Diphenyl-2-[3-(1-methylimidazol-2-ylthio)-2-hydroxypropylthio]imidazol
BA 4,5-Diphenyl-2-[3-(1-methylimidazol-2-ylthio)propylthio]imidazol-dimaleat
BB 4,5-Diphenyl-2-[3-(3-amino-1,2,4-triazol-5-ylthio)propylthio]imidazol
BC 4,5-Diphenyl-2-[3-(pyrid-2-ylthio)propylthio]imidazol
BD E-4,5-Diphenyl-2-[4-(1-methylimidazol-2-ylthio)but-2-enylthio]imidazol
BE 4,5-Diphenyl-2-[3-(pyrimidin-2-ylsulfonyl)-propylthio]imidazol
BF 4,5-Diphenyl-2-[4-(pyrimidin-2-ylsulfonyl)-butylthio]imidazol
BG 4,5-Diphenyl-2-[3-pyrid-2-ylsulfinyl)propylthio]imidazol
BH 4,5-Diphenyl-2-[4-(pyrimidin-2-ylthio)butylthio]imidazol
BI 1,6-Bis(4,5-diphenylimidazol-2-ylthio)hexan
BJ 4,5-Bis(4-methylphenyl)-2-[3-(1-methylimidazol-2-ylthio)propylthio]imidazol
BK 4,5-Bis(4-methoxyphenyl)-2-[3-(pyrimidin-2-ylsulfonyl)propylthio]imidazol
BL 4,5-Diphenyl-2-[4-(imidazol-2-ylthio)butylthio]imidazol
BM 4,5-Diphenyl-2-[3-(pyrid-4-ylsulfonyl)propylthio]imidazol
BN 4,5-Diphenyl-2-[3-(pyrid-2-ylsulfonyl)propylthio]imidazol
BO 4,5-Diphenyl-2-[4-(pyrid-2-ylthio)butylthio]imidazol
BP 1,5-Bis(4,5-diphenylimidazol-2-ylthio)pentan
BQ 1,4-Bis(4,5-diphenylimidazol-2-ylthio)but-2-in
BR 4,5-Diphenyl-2-[4-(1-methylimidazol-2-ylthio)but-2-inylthio]imidazol
BS 4,5-Bis(4-fluorphenyl)-2-[3-(1-methylimidazol-2-ylthio)propylthio]imidazol
BT 4,5-Bis(4-methoxyphenyl)-2-[4-(pyrimidin-2-ylsulfonyl)butylthio]imidazol
BU 4,5-Diphenyl-2-[4-(pyrid-2-ylsulfonyl)butylthio]imidazol
BV 4,5-Diphenyl-2-[4-(pyrid-2-ylsulfinyl)butylthio]imidazol
BW 4,5-Diphenyl-2-{3-[4(5)-methylimidazol-2-ylthio]propylthio}imidazol
BX 4,5-Diphenyl-2-[5-(imidazol-2-ylthio)pentylthio]imidazol
BY 4,5-Diphenyl-2-[3-(imidazol-2-ylthio)propylthio]imidazol-dihydrochlorid
BZ 4,5-Diphenyl-2-[3-(imidazol-2-ylthio)propylthio]imidazol-dimaleat
CA Z-1,4-Bis(4,5-diphenylimidazol-2-ylthio)-but-2-en
CB 4,5-Diphenyl-2-[4-(pyrid-2-ylsulfonyl)butylthio]imidazol-hydrochlorid
CC 4,5-Diphenyl-2-[4-(imidazol-2-ylsulfonyl)butylthio]imidazol.

9. Verfahren zur Herstellung einer Verbindung der allgemeinen Formel I gemäß der Definition in Anspruch 5 durch
A) wenn A, R¹, R², b und Het der Definition in Anspruch 5 genügen und a 0 ist, Umsetzen eines Salzes der allgemeinen Formel: 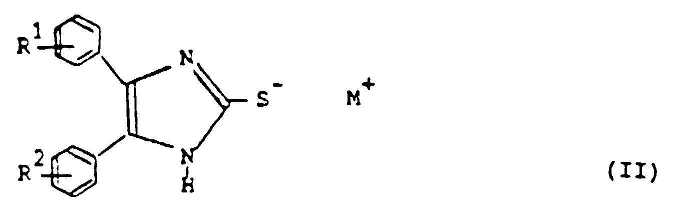 worin R¹ und R² die in Anspruch 5 angegebene Bedeutung besitzen und M für ein Alkalimetallatom steht, mit einer Verbindung der Formel:
Z¹-A-S(O)_{b}-Het (III)
worin A, b und Het die in Anspruch 5 angegebene Bedeutung besitzen und Z¹ für ein Chlor-, Brom- oder Jodatom oder eine Alkyl- oder Arylsulfonyloxygruppe steht;
B) wenn A, R¹, R², a und Het der Definition in Anspruch 5 genügen und b 0 ist, Umsetzen eines Salzes der Formel:
Het-S⁻ M⁺ (V)
worin Het und M die oben angegebene Bedeutung besitzen, mit einer Verbindung der allgemeinen Formel: 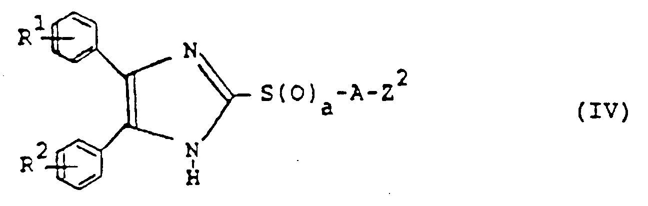 worin A, R¹, R² und a die in Anspruch 5 angegebene Bedeutung besitzen und Z² für ein Chlor-, Brom- oder Jodatom oder eine Alkyl- oder Arylsulfonyloxygruppe steht;
C) wenn die Verbindung der Formel I der folgenden allgemeinen Formel entspricht: 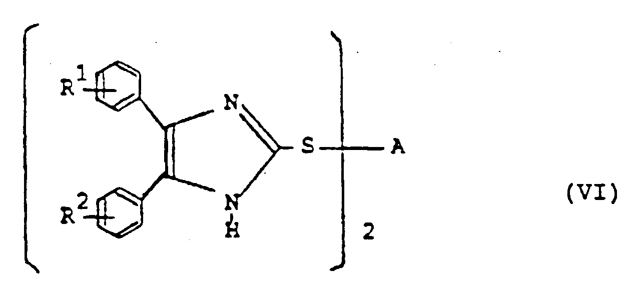 worin A, R¹ und R² der Definition in Anspruch 5 genügen, Umsetzen eines Salzes der Formel II mit einer Verbindung der Formel:
Z¹-A-Z² (VII)
worin A, Z¹ und Z² die oben angegebene Bedeutung besitzen;
D) wenn A, R¹, R² und Het der Definition in Anspruch 5 genügen und a und b beide 0 sind, Umsetzen eines Salzes der Formel II und eines Salzes der Formel (V) mit einer Verbindung der Formel (VII), wobei die verschiedenen Symbole die oben angegebene Bedeutung besitzen;
E) wenn A, R¹, R² und Het die oben angegebene Bedeutung besitzen und mindestens einer der Indices a und b 1 oder 2 bedeutet, Oxidieren einer Verbindung der Formel I, worin A, R¹ und Het die oben angegebene Bedeutung besitzen und a und/oder b kleiner als im gewünschten Produkt ist (sind);
gegebenenfalls anschließende Umwandlung einer Verbindung der allgemeinen Formel I in eine andere Verbindung der allgemeinen Formel I und
gegebenenfalls anschließende Umwandlung einer Verbindung der allgemeinen Formel I in ein pharmazeutisch akzeptables Salz hiervon.

10. Pharmazeutische Zubereitung, die ein Imidazolderivat der Formel I gemäß der Definition in Anspruch 3 oder ein pharmazeutisch akzeptables Salz hiervon in Verbindung mit einem pharmazeutisch akzeptablen Träger oder Überzug umfaßt.

## Revendications

1. Dérivé d'imidazole de formule générale : 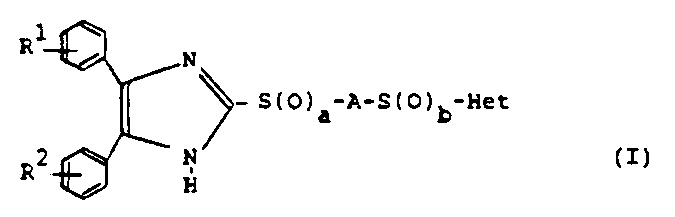 dans laquelle :
A représente un groupe méthylène ou un groupe -CH₂-A'-CH₂- dans lequel A' représente une liaison directe, un groupe alcanediyle, alcènediyle ou alcynediyle linéaire jusqu'en C₁₄, ou un groupe hydroxyméthylène, ou bien A' représente un groupe phénylène facultativement substitué par 1 à 4 substituants choisis parmi les atomes d'halogènes, les groupes alkyles (facultativement substitués par un ou plusieurs atomes d'halogènes), les groupes alcoxy, alkylthio, alkylamino, carboxy et alcoxycarbonyles ;
R¹ et R² sont identiques ou différents et représentent chacun un atome d'hydrogène ou d'halogène, un groupe alkyle (facultativement substitué par un ou plusieurs atomes d'halogènes) ou un groupe alcoxy, alkylthio, alkylamino, carboxy ou alcoxycarbonyle ;
a et b sont identiques ou différents et représentent chacun 0, 1 ou 2 ; et
Het représente un groupe hétérocyclique contenant 5 à 7 atomes cycliques choisis parmi les atomes de carbone, d'azote, de soufre et d'oxygène, facultativement substitué par 1 ou 2 substituants choisis indépendamment parmi : les atomes d'halogènes, les groupes alkyles (facultativement substitués par un ou plusieurs atomes d'halogènes), les groupes hydroxy, amino, alcoxy, alkylthio, alkylamino, carboxy, alcoxycarbonyles et pyridyles et les groupes phényles [facultativement substitués par un ou plusieurs subsituants choisis parmi les atomes d'halogènes, les groupes alkyles (facultativement substitués par un ou plusieurs atomes d'halogènes) et les groupes alcoxy, alkylthio, alkylamino, carboxy et alcoxycarbonyles] ;
et leurs sels acceptables en pharmacie ;
dans lesquels tous les groupes et restes alkyles, sauf autre indication, sont à chaîne droite ou ramifiée en C₁-C₅ ; et les atomes d'halogènes, sauf autre indication, sont des atomes de fluor, de chlore, de brome ou d'iode ;
pour l'utilisation dans la préparation d'une composition pharmaceutique pour le traitement d'un état qui peut être amélioré par administration d'un inhibiteur de l'acyl-coenzyme A : la cholestérol-O-acyltransférase.

2. Composé selon la revendication 1 pour l'utilisation dans le traitement de l'athérosclérose, de l'hyperlipidémie, des maladies du stockage des esters de cholestérol ou de l'athérome dans les greffes de veines.

3. Dérivé d'imidazole de formule générale I selon la revendication 1 ou un de ses sels acceptables en pharmacie, avec la condition que, lorsque R¹ et R² sont identiques et représentent chacun un atome de fluor, de chlore ou de brome ou un groupe trifluorométhyle, méthoxy ou méthylthio en position para et A représente un groupe méthylène, 1,2-éthanediyle, 1,3-propanediyle, 2-hydroxy-1,3-propanediyle ou 1,4-butanediyle, Het représente alors un groupe autre qu'un groupe de formule générale : 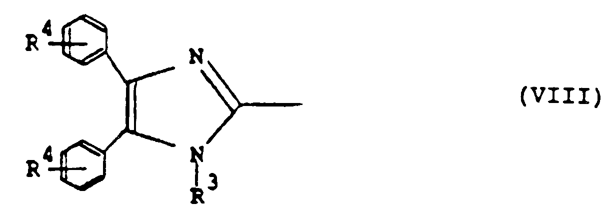 dans laquelle R³ représente un atome d'hydrogène ou un groupe méthyle et les symboles R⁴ sont identiques et représentent chacun un atome de fluor, de chlore ou de brome ou un groupe trifluorométhyle, méthoxy ou méthylthio en position para, pour l'utilisation dans une méthode de traitement du corps humain ou animal par thérapie.

4. Composé selon la revendication 3 pour l'utilisation dans une méthode de traitement du corps humain ou animal par thérapie de l'athérosclérose, de l'hyperlipidémie, des maladies du stockage des esters de cholestérol ou de l'athérome dans les greffes de veines.

5. Dérivé d'imidazole de formule générale I, tel que défini dans la revendication 1 ou un de ses sels acceptables en pharmacie, avec les conditions suivantes :
(1) lorsque R¹ et R² sont identiques et représentent chacun un atome de fluor, de chlore ou de brome ou un groupe trifluorométhyle, méthoxy ou méthylthio en position para et A représente un groupe méthylène, 1,2-éthanediyle, 1,3-propanediyle, 2-hydroxy-1,3-propanediyle ou 1,4-butanediyle, Het représente alors un groupe autre qu'un groupe de formule VIII, dans laquelle R³ représente un atome d'hydrogène ou un groupe méthyle et les symboles R⁴ sont identiques et représentent chacun un atome de fluor, de chlore ou de brome ou un groupe trifluorométhyle, méthoxy ou méthylthio en position para ; et
(2) lorsque A représente un groupe 1,3-propanediyle ou 2-hydroxy-1,3-propanediyle, R¹ et R² représentent tous deux des atomes d'hydrogène et a et b représentent tous deux zéro, Het représente alors un groupe autre qu'un groupe de formule VIII dans laquelle R³ représente un atome d'hydrogène et les symboles R⁴ représente tous deux des atomes d'hydrogène.

6. Composé selon la revendication 5 dans lequel l'un au moins des symboles a une valeur choisie parmi les suivantes :
(i) A représente un groupe -CH₂-A'-CH₂- dans lequel A' représente une liaison directe, un groupe alcanediyle, alcènediyle ou alcynediyle linéaire jusqu'en C₇ ou un groupe hydroxyméthylène ou un groupe phénylène substitué ou non ;
(ii) R¹ et R² sont identiques ou différents et représentent chacun un atome d'hydrogène ou d'halogène ou un groupe alkyle ou alcoxy ; et
(iii) Het représente un groupe hétérocyclique à 5 ou 6 chaînons facultativement substitué contenant jusqu'à 4 atomes d'azote, substitué ou non par alkyle, amino, pyridyle ou phényle facultativement substitué.

7. Composé selon la revendication 5 ou 6 dans lequel l'un au moins des symboles a une valeur choisie parmi les suivantes :
(i) A' représente un groupe méthylène ou un groupe alcanediyle linéaire contenant 2, 3, 4 ou 7 atomes de carbone ou un groupe vinylène ou éthynylène ou un groupe phénylène non substitué ;
(ii) R¹ et R² sont identiques ou différents et représentent chacun un atome d'hydrogène ou de fluor ou un groupe méthyle ou méthoxy en position 4 ; et
(iii) Het représente un groupe pyrimidine-2-yle, pyridine-2-yle, pyridine-4-yle, imidazole-2-yle, 1,2,4-triazole-3-yle ou tétrazole-5-yle non substitué ou substitué par méthyle, amino, pyridine-2-yle ou phényle facultativement substitué.

8. Composé selon la revendication 1 qui est choisi parmi les suivants :
A 2-[3-(pyrimidine-2-ylthio)propylthio)-4,5-diphénylimidazole
B 2-[3-(pyridine-4-ylthio)propylthio]-4,5-diphénylimidazole
C 2-[3-(1-méthylimidazole-2-ylthio)propylthio]-4,5-diphénylimidazole
D dichlorhydrate de 2-[3-(1-méthylimidazole-2-ylthio)propylthio]-4,5-diphénylimidazole
E 1,2-bis(4,5-diphénylimidazole-2-ylthio)éthane
G 2-[3-(4,6-diméthylpyrimidine-2-ylthio)propylthio]-4,5-diphénylimidazole
H 2-[3-(1-méthylimidazole-2-ylsulfonyl)propylthio]-4,5-diphénylimidazole
I 2-[3-(4-méthyl-1,2,4-triazole-3-ylthio)propylthio]-4,5-diphénylimidazole
J 2-[3-(1-méthyltétrazole-5-ylthio)propylthio]-4,5-diphénylimidazole
K dichlorhydrate de 1-(4,5-diphénylimidazole-2-ylthio)-3-[(4,5-dipyridine-2-yl)imidazole-2-ylthio]propane
L 2-[3-(1-méthyltétrazole-5-ylsulfonyl)propylthio]-4,5-diphénylimidazole
M 2-[3-(imidazole-2-ylthio)propylthio]-4,5-diphénylimidazole
N 2-[4-(1-méthylimidazole-2-ylthio)butylthio]-4,5-diphénylimidazole
O 2-[4-(1-méthylimidazole-2-ylsulfonyl)butylsulfonyl]-4,5-diphénylimidazole
P 1,4-bis(4,5-diphénylimidazole-2-ylthio)butane
Q E-1,4-bis(4,5-diphénylimidazole-2-ylthio)-2-butène
R 2-[6-(1-méthylimidazole-2-ylthio)hexylthio]-4,5-diphénylimidazole
S 1,9-bis(4,5-diphénylimidazole-2-ylthio)nonane
T 2-[9-(1-méthylimidazole-2-ylthio)nonylthio]-4,5-diphénylimidazole
AA 1,2-bis(4,5-diphénylimidazole-2-ylthiométhyl)benzène
AB 1,3-bis(4,5-diphénylimidazole-2-ylthiométhyl)benzène
AC 1,4-bis(4,5-diphénylimidazole-2-ylthiométhyl)benzène
AE 4,5-diphényl-2-[3-(1-méthylimidazole-2-ylthio)-2-hydroxypropylthio]imidazole
BA dimaléate de 4,5-diphényl-2-[3-méthylimidazole-2-ylthio)propylthio]imidazole
BB 4,5-diphényl-2-[3-(3-amino-1,2,4-triazole-5-ylthio)propylthio]imidazole
BC 4,5-diphényl-2-[3-(2-pyridylthio)propylthio]-imidazole
BD E-4,5-diphényl-2-[4-(1-méthylimidazole-2-ylthio)énylthio]imidazole
BE 4,5-diphényl-2-[3-(pyrimidine-2-ylsulfonyl)-propylthio]imidazole
BF 4,5-diphényl-2-[4-(pyrimidine-2-ylsulfonyl)-butylthio]imidazole
BG 4,5-diphényl-2-[3-(2-pyridylsulfonyl)propylthio]imidazole
BH 4,5-diphényl-2-[4-(pyrimidine-2-ylthio)butylthio]imidazole
BI 1,6-bis(4,5-diphénylimidazole-2-ylthio)hexane
BJ 4,5-bis(4-méthylphényl)-2-[3-(1-méthylimidazole-2-ylthio)propylthio]imidazole
BK 4,5-bis(4-méthoxyphényl)-2-[3-(pyrimidine-2-yl-sulfonyl)propylthio]imidazole
BL 4,5-diphényl-2-[4-(imidazole-2-ylthio)butylthio]imidazole
BM 4,5-diphényl-2-[3-(4-pyridylsulfonyl)propylthio]imidazole
BN 4,5-diphényl-2-[3-(2-pyridylsulfonyl)propylthio]imidazole
BO 4,5-diphényl-2-[4-(2-pyridylthio)butylthio]imidazole
BP 1,5-bis(4,5-diphénylimidazole-2-ylthio)pentane
BQ 1,4-bis(4,5-diphénylimidazole-2-ylthio)butyne-2
BR 4,5-diphényl-2-[4-(1-méthylimidazole-2-ylthio)-2-butynylthio]imidazole
BS 4,5-bis(4-fluorophényl)-2-[3-(1-méthylimidazole-2-ylthio)propylthio]imidazole
BT 4,5-bis(4-méthoxyphényl)-2-[4-(pyrimidine-2-yl-sulfonyl)butylthio]imidazole
BU 4,5-diphényl-2-[4-(2-pyridylsulfonyl)butylthio]imidazole
BV 4,5-diphényl-2-[4-(2-pyridylsulfonyl)butylthio]imidazole
BW 4,5-diphényl-2-{3-[4(5)-méthylimidazole-2-ylthio]propylthio)imidazole
BX 4,5-diphényl-2-[5-imidazole-2-ylthio)pentylthio]imidazole
BY dichlorhydrate de 4,5-diphényl-2-[3-(imidazole-2-ylthio)propylthio]imidazole
BZ dimaléate de 4,5-diphényle-2-[3-(imidazole-2-ylthio)propylthio]imidazole
CA Z-1,4-bis(4,5-diphénylimidazole-2-ylthio)-2-butène
CB chlorhydrate de 4,5-diphényl-2-[4-(2-pyridylsulfonyl)butylthio]imidazole et
CC 4,5-diphényl-2-[4-(imidazole-2-ylsulfonyl)butylthio]imidazole

9. Procédé pour la préparation d'un composé de formule générale (I) telle que définie dans la revendication 5 qui comprend :
(A) lorsque A, R¹, R², b et Het sont tels que définis dans la revendication 5 et a représente zéro, la réaction d'un sel de formule générale : 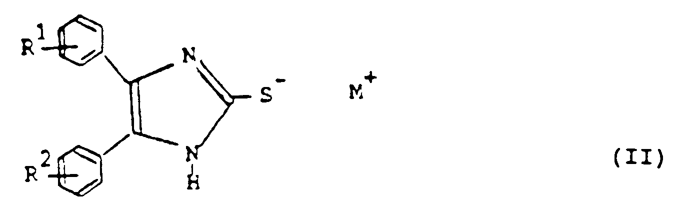 dans laquelle R¹ et R² sont tels que définis dans la revendication 5 et M représente un atome de métal alcalin, avec un composé de formule :
Z¹-A-S(O)_{b}-Het (III)
dans laquelle A, b et Het sont tels que définis dans la revendication 5 et Z¹ représente un atome de chlore, de brome ou d'iode ou un groupe alkyl- ou arylsulfonyloxy ;
(B) lorsque A, R¹, R², a et Het sont tels que définis dans la revendication 5 et b représente zéro, la réaction d'un sel de formule :
Het-S⁻ M⁺ (V)
dans laquelle Het et M sont tels que définis précédemment, avec un composé de formule générale : 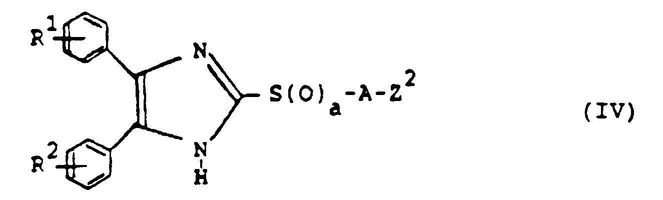 dans laquelle A, R¹ R² et a sont tels que définis dans la revendication 5 et Z² représente un atome de chlore, de brome ou d'iode ou un groupe alkyl- ou arylsulfonyloxy ;
(C) lorsque le composé de formule (I) répond à la formule générale : 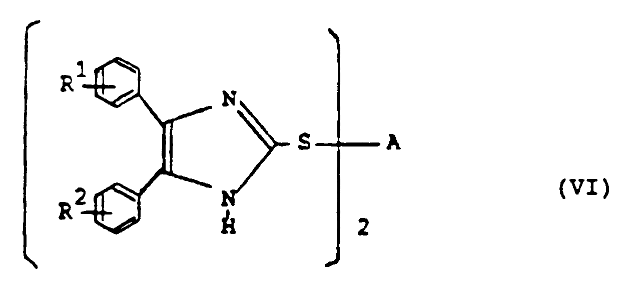 dans laquelle A, R¹ et R² sont tels que définis dans la revendication 5, la réaction d'un sel de formule (II) avec un composé de formule :
Z¹-A-Z² (VII)
dans laquelle A, Z¹ et Z² sont tels que définis précédemment ;
(D) lorsque A, R¹, R² et Het sont tels que définis dans la revendication 5 et a et b sont tous deux égaux à zéro, la réaction d'un sel de formule (II) et d'un sel de formule (V) avec un composé de formule (VII), dans lesquelles les divers symboles sont tels que définis précédemment ;
(E) lorsque A, R¹, R² et Het sont tels que définis précédemment et l'un au moins des symboles a et b représente 1 ou 2, l'oxydation d'un composé de formule (I) dans laquelle A, R¹ et Het sont tels que définis précédemment et a et/ou b est inférieur à sa valeur dans le produit désiré ;
suivie facultativement de la conversion d'un composé de formule générale (I) en un autre composé de formule générale (I) ;
suivie facultativement de la conversion d'un composé de formule générale (I) en un de ses sels acceptables en pharmacie.

10. Composition pharmaceutique qui comprend un dérivé d'imidazole de formule (I) telle que définie dans la revendication 3 ou un de ses sels acceptables en pharmacie, en association avec un support ou un revêtement acceptable en pharmacie.
